# EUROPÄISCHE PATENTSCHRIFT

(11) **EP 1 240 333 B1**
(45) Veröffentlichungstag und Bekanntmachung des Hinweises auf die Patenterteilung: **09.04.2008**
(21) Anmeldenummer: 00987164.1
(22) Anmeldetag: 16.11.2000
(51) Int. Cl.: C12N 15/49

(54) **DAS GENOM DES HIV-1 INTERSUBTYPS (C/B') UND SEINE ANWENDUNGEN**
THE GENOME OF THE HIV-1 INTER-SUBTYPE (C/B') AND USE THEREOF
GENOME DE L'INTER-SOUS-TYPE VIH-1 (C/B') ET SES APPLICATIONS

(30) Priorität: 16.11.1999 DE 19955089
(43) Veröffentlichungstag der Anmeldung: 18.09.2002
(73) Patentinhaber: GENEART AG, 93053 Regensburg (DE); Shao, Yiming, Beijing Xuan Wu Qu 1000050 (CN)
(72) Erfinder: SHAO, Yiming, Beijing Xuan Wu Qu 1000050 (CN); WAGNER, Ralf, 93949 Regensburg (DE); WOLF, Hans, 82319 Starnberg (DE); GRAF, Marcus, 93047 Regensburg (DE)
(74) Vertreter: Dehmel, Albrecht
(86) Internationale Anmeldenummer: PCT/DE2000/004073
(87) Internationale Veröffentlichungsnummer: WO 2001/036614

(56) Entgegenhaltungen:
- EP-A- 0 736 600
- WO-A-00/39304
- WO-A-99/41397
- GRAF M ET AL: "Cloning and characterization of a virtually full-length HIV type 1 genome from a subtype B'-Thai strain representing the most prevalent B-clade isolate in China." AIDS RESEARCH AND HUMAN RETROVIRUSES, Bd. 14, Nr. 3, 10. Februar 1998 (1998-02-10), Seiten 285-288, XP000999769 ISSN: 0889-2229
- CHOI, DJ ET AL: "HIV type 1 isolate Z321, the strain used to make a therapeutic HIV type 1 immunogen, is intersubtype recombinant" AIDS RES HUM RETROVIRUSES, Bd. 13, Nr. 4, 1. März 1997 (1997-03-01), Seiten 357-361, XP000999770
- SIEPEL, AC ET AL: "A computer program designed to screen rapidly for HIV type 1 intersubtype recombinant sequences" AIDS RES HUM RETROVIRUSES, Bd. 11, Nr. 11, November 1995 (1995-11), Seiten 1413-1416, XP000999771
- SU, L. ET AL.: "Characterization of a virtually full-length human immunodeficiency virus type 1 genome of a prevalent intersubtype (C/B') recombinant strain in China" JOURNAL OF VIROLOGY, Bd. 74, Nr. 23, Dezember 2000 (2000-12), Seiten 11367-11376, XP000999818

### SEQUENZPROTOKOLL

<110> Geneart GmbH
<120> Das Genom des HIV-1 Intersubtyps (C/B') und seine Anwendungen
<130> WAG-001 PCT
<140> xx
   <141> 2000-11-16
<150> DE 199 55 089.1
   <151> 1999-11-16
<160> 3
<170> PatentIn Ver. 2.1
<210> 1
   <211> 9078
   <212> DNA
   <213> Human immunodeficiency virus
<400> 1
   aatctctagc agtggcgccc gaacagggac ttgaaagcga aagtaagacc agaggagatc 60
   tctcgacgca ggactcggct tgctgaagtg cactcggcaa gaggcgagag cggcgactgg 120
   tgagtacgcc aattatattt gactagcgga ggctagaagg agagagatgg gtgcgagagc 180
   gtcaatatta agagggggaa aattagataa atgggaaaaa attaggttaa ggccaggggg 240
   aaagaaacac tatatgctaa aacacctagt atgggcaagc agggagctgg aaagatttgc 300
   acttaaccct ggccttttag agacatcaga aggctgtaaa caaataatga aacagctaca 360
   atcagctctt cagacaggaa cagaggaact tagatcatta ttcaacacag tagcaactcc 420
   ctattgtgta catacagaga tagatgtacg agacaccaga gaagccttag acaagataga 480
   ggaagaacaa aacaaaattc agcaaaaaac acagcaggca aaggaggctg acgggaaggt 540
   cagtcaaaat tatcctatag tacagaatct ccaagggcaa atggtacatc agcccatatc 600
   acctagaact ttaaatgcat gggtaaaagt ggtagaagag aaggctttta gcccagaagt 660
   aatacccatg ttttcagcgt tatcagaagg agccacccca caagatttaa acaccatgct 720
   aaacacagtg gggggacatc aagcagctat gcaaatatta aaagatacca tcaatgaaga 780
   ggctgcagaa tgggatagat tacatccagt acatgcaggg cctattgcac caggccaaat 840
   gagagaacca aggggaagtg acatagcagg aactactagt aacctacagg aacaaatagc 900
   atggatgacg agtaacccac ctgttccagt aggagacatc tataaaagat ggataattct 960
   gggattaaat aaaatagtaa gaatgtatag ccctaccagc attctggaca taaaacaagg 1020
   gccaaaggaa ccctttagag actatgtaga ccggttcttt aaaactttaa gagcggaaca 1080
   agctacgcaa ggtgtaaaaa attggatgac agacaccttg ttggtccaaa atgcgaaccc 1140
   agattgtaag accattttaa gagcattagg accaggggct tcaatagaag aaatgatgac 1200
   agcatgtcag ggagtgggag gacctagcca taaagcaaaa gtgttggccg aggcaatgag 1260
   ccaaacaaac agtgccatac tgatgcagag aagcaatttt aaaggctcta aaagaattgt 1320
   taaatgtttc aactgtggca aggaagggca catagccaga aattgcaggg cccctaggaa 1380
   aaagggctgt tggaaatgtg gaaaagaagg acaccaaatg aaagattgta ctgagagaca 1440
   ggccaatttt ttagggaaaa tctggccctc ccacaaggga gggccaggga attttcttca 1500
   gaacagacca gagccaacag ccccaccaga ggagagcttc aggtttgggg aagagacaac 1560
   aactccatct cagaagcagg agccaataga caaggaacta tatcctttaa cttccctcaa 1620
   atcactcttt ggcaacgacc cctcgtcaca ataaagatag gggggcaatt aaaggaagct 1680
   ctattagata caggagcagg tgatacagta ttagaagacc tgaatttgcc agggaaatgg 1740
   aaaccaaaaa tgataggggg aattggaggt tttatcaaag taagacagta tgaacagata 1800
   cccatagaaa tttgcggaca caaagctata ggtacagtat tagtaggacc tacacctgtc 1860
   aacataattg gaagaaatct gttgactcag cttggttgca ctttaaattt tccaatcagt 1920
   cccattgaaa ctgtaccagt aaaattaaag ccaggaatgg atggcccaaa ggttaaacaa 1980
   tggccattga cagaagagaa aataaaagca ttaacagcaa tttgtgatga aatggagaaa 2040
   gaaggaaaaa ttacaaaaat tgggcctgaa aatccatata acactccaat atttgccata 2100
   aaaaagaagg acagtactaa gtggagaaag ttagtagatt tcagggaact caataaaaga 2160
   actcaagatt tttgggaagt tcaattagga ataccacacc cagcagggtt aaaaaagaaa 2220
   aaatcagtga cagtactgga tgtgggggat gcatattttt caattccttt atatgaagac 2280
   ttcaggaagt atactgcatt caccatacct agtagaaaca atgaaacacc agggattagg 2340
   tatcagtaca atgtacttcc acagggatgg aaaggatcac tagcaatatt ccaaagtagc 2400
   atgacaaaaa ccttagagcc ttttagaaaa caaaatccag gcatagttat ctatcaatac 2460
   atggatgatt tgtatgtagg atctgactta gagatagggc agcatagaac aaaaatagag 2520
   gaactgagac aacatttgtt gaggtgggga tttaccacac cagacaagaa acattagaaa 2580
   gaacctccat ttctttggat ggggtatgaa ctccatcctg acaaatggac agtacagcct 2640
   acacagctgc cagaaaaaga tagctggact gtcaatgata tacaaaagtt agtgggaaaa 2700
   ttaaactggg caagtcagat ttatcctgga attaaagtaa ggcaactttg taaactcctt 2760
   aggggggcca aagcactaac agacatagta ccactaactg aagaagcaga attagaattg 2820
   gcagaaaaca gggaaattct aaaagaacca gtacatggag tatactatga cccatcaaaa 2880
   gacttgatag ctgaaataca gaaacagggg caggaacaat ggacatatca aatttaccaa 2940
   gaaccattca aaaatctaaa aacagggaag tatgcaaaaa tgaggactgc ccacactaat 3000
   gatgtaaaac aattaacaga ggctgtgcag aaaatagcca tggaaggcat agtaatatgg 3060
   ggaaaaactc ctaaatttag attacccatc caaaaagaaa catgggagac atggtggaca 3120
   gactattggc aagccacctg gattcctgag tgggaatttg ttaatacccc tcccttagta 3180
   aaattatggt accagctgga aaaagatccc atagtaggag tagaaacttt ctatgtagat 3240
   ggagcagcta atagggagac taaaatagga aaagcagggt atgttactga cagaggaagg 3300
   aagaaaattg tttctctaac tgaaacaaca aatcagaaga ctgaattgca agcaatttgt 3360
   atagctttgc aagattcagg atcagaagta aacatagtaa cagattcaca gtatgcatta 3420
   gggatcattc aagcacaacc agataagagt gaatcagagt tagttaacca aataatagaa 3480
   caattaatga aaaaggaaag agtctacctg tcatgggtac cagcacataa aggaattgga 3540
   ggaaatgaac aagtagataa attagtaagt agtggaatca ggaaagtgct atttctagat 3600
   ggaatagata aagctcaaga agagcatgaa aagtatcaca gcaattggag agcaatggct 3660
   agtgacttta atctgccacc catagtagca aaagaaatag tggctagctg tgatcaatgt 3720
   cagctaaaag gagaagccat gcatggacaa gtagactgta gtccagggat atggcaatta 3780
   gattgtacac atttagaagg aaaaatcatc ctggtagcag tccatgtagc cagtggctac 3840
   atggaagcag aggttatccc agcagaaaca ggacaagaga cagcatactt tatactaaaa 3900
   ttagcaggaa gatggccagt caaagtaata catacagata atggtagtaa tttcaccagt 3960
   actgcagtta aggcagcctg ttggtgggca ggtatccaac aggaatttgg aattccctac 4020
   agtccccaaa gtcagggagt agtagaagcc atgaataaag aattaaagaa aattataggg 4080
   caggtaagag atcaagctga gcaccttaag acagcagtac taatggcagt attcattcac 4140
   aattttaaaa gaaaaggggg gattgggggg tacagtgcag gggaaagaat aatagatata 4200
   atagcaacag acatacaaac taaagaatta caaaaacaga ttacaaaaat tcaaaatttt 4260
   cgggtttatt acagagacag cagagacccc agttggaaag gaccagccaa actactctgg 4320
   aaaggtgaag gggcagtaat aatacaagat aatagtgaca taaaggtagt accaaggagg 4380
   aaagcaaaaa tcattaagga ctatggaaaa cagatggcag gtgctgattg tgtggcaggt 4440
   agacaggatg aagattagaa catggaatag tttagtaaaa caccatatgt atgtttcaag 4500
   gagagctaat ggatggtttt acagacatca ttatgacagc agacatccaa aagtaagttc 4560
   agaagtacac atcccattag gaaaggctaa attagtaata aaaacatatt gggggttgca 4620
   gacaggagaa agagatcggc atttgggtca tggagtctcc atagaatgga gattgagaag 4680
   atataccaca caaatagaac ctggcctggc agaccagcta attcatttgt attattttga 4740
   ttgttttgca gactctgata taaggaaagc catattagga cacatagtta ttcctaggtg 4800
   tgactatcaa gcaggacata ataataaggt aggatctcta caatacttgg cactgacagc 4860
   attgataaaa ccaaaaaaga taaagccacc tctgcctagt atcaagaaat tagtagagga 4920
   tagatggaac aatccccagg agatcagggg ccgcagaggg aaccacacaa tgaatggaca 4980
   ctagagcttc tagaggagct caagcaggaa gctgttagac actttcctag accatggctt 5040
   catagcttag gacaacatat ctatgaaaca tatggggata cttgggcagg agtggaagcc 5100
   ataataagaa ttctgcaaca actgctgttt attcatttca gaattgggtg tcagcatagc 5160
   agaataggca ttttgagaca gagaagaaca agaaatggag ccagtaaatc ataaattaga 5220
   gccttgggag catccaggaa gtcagcctaa gactgcttgt aacagttgct attgtaaaaa 5280
   gtgctgcttt cattgccaag tttgtttcac gaaaaaaggc ttaggcatct tctatggcag 5340
   gaagaagcga agacagcgac gaagcgctca tcgaagcagt gaggatcatc aaaatcctat 5400
   atcaaagcag taagtagtaa atgtaatgca agctttaacc attttagcaa tagtagcctt 5460
   agtagtagca acaataatag caatagttgt gtggaccata gtattcatag aatataggaa 5520
   aatattaaga cagaaaaaaa tagacaggtt aattgataga ataagagaaa gagcagaaga 5580
   cagtggcaat gagggtgacg gggatcagga agaattatcg gcatttatgg agatggggca 5640
   ccatgctcct tgggatgttg atgatcagta gtgctgtagg aaacttgtgg gtcacagtct 5700
   attatggggt acctgtatgg aaaggggcaa ccaccacttt attttgtgca tcagatgcta 5760
   aagcatatga tacagaggta cataatgttt gggctacaca tgcctgtgta cccgcagacc 5820
   ccaacccaca agaaatggtt ttggaaaatg taacagaaaa ttttaacatg tggaaaaatg 5880
   aaatggtaaa tcagatgcag gaagatgtaa tcagtttatg ggatcaaagc ctaaaaccat 5940
   gtgtaaagtt gaccccactc tgtgtcactt tagaatgtag aaatgttagc agtaatagta 6000
   atgataccta ccatgagacc taccatgaga gcatgaagga aatgaaaaat tgctctttca 6060
   atgcaaccac agtagtaaga gataggaagc agacagtgta tgcacttttt tatagacttg 6120
   atatagtacc acttactaag aagaactata gtgagaattc tagtgagtat tatagattaa 6180
   taaattgtaa tacctcagcc ataacacaag cctgtccaaa ggtcactttt gatccaattc 6240
   ctatacacta ttgcactcca gctggttatg caattctaaa gtgtaatgat aagatattca 6300
   atgggacagg accatgccat aatgttagca cagtacaatg tacacatggg attaagccag 6360
   tggtatcaac tcaactactg ttaaatggta gcctagcaga aggagaaata ataattagat 6420
   ctgaaaatct gacaaacaat gtcaaaacaa taatagtaca tcttaatcaa tctgtagaaa 6480
   ttgtatgtac aagacccggc aataatacaa gaaaaagtat aaggatagga ccaggacaaa 6540
   cattctatgc aacaggagac ataataggag acataagaca agcacattgt aacattagtg 6600
   aagataaatg gaatgaaact ttacaaaggg taagtaaaaa attagcagaa cacttccaga 6660
   ataaaacaat aaaatttgca tcatcctcag gaggggacct agaagttaca acacatagct 6720
   ttaattgtag aggagaattt ttctattgta atacatcagg cctgtttaat ggtgcataca 6780
   cgcctaatgg tacaaaaagt aattcaagct caatcatcac aatcccatgc agaataaagc 6840
   aaattataaa tatgtggcag gaggtaggac gagcaatgta tgcccctccc ataaaaggaa 6900
   acataacatg taaatcaaat atcacaggac tactattggt acgtgatgga ggaacagagc 6960
   caaatgatac agagacattc agacctggag gaggagatat gaggaacaat tggagaagtg 7020
   aattatataa atataaagtg gtagaaatta agccattggg agtagcaccc actacaacaa 7080
   aaaggagagt ggtggagaga gaaaaaagag cagtgggaat aggagctgtg ttccttgggt 7140
   tcttaggagt agcaggaagc actatgggcg cggcgtcaat aacgctgacg gtacaggcca 7200
   gacaattgct gtctggtata gtgcaacagc aaagcaattt gctgagggct atagaagcgc 7260
   aacagcatct gttgcaactc acggtctggg gcattaagca gctccagaca agagtcctgg 7320
   ctatagaaag atacctaaag gatcaacagc tcctagggat ttggggctgc tctggaaaac 7380
   tcatctgcac tactgctgta ccttggaact ccagttggag taacaaatct caaaaagaga 7440
   tttgggataa catgacctgg atgcaatggg ataaagaaat tagtaattac acaaacacag 7500
   tatacaggtt gcttgaagaa tcgcaaaacc agcaggaaag gaatgaaaaa gatctattag 7560
   cattggacag ttggaaaaat ctatggagtt ggtttgacat aacaaattgg ctgtggtata 7620
   taaaaatatt cataataata gtaggaggct tgataggttt aagaataatt tttgctgtgc 7680
   tctctatagt aaatagagtt aggcagggat actcaccttt gtcgtttcag acccttaccc 7740
   cgaacccagg gggacccgac aggctcggaa gaatcgaaga agaaggtgga aagcaagaca 7800
   gggacagatc cattcgatta gtgaacggat tcttagcgct tgcctgggac gacctgcgga 7860
   acctgtgcct cttcagctac caccgattga gggacttcac attagtggca gcgagggtgg 7920
   tggaacttct gggacgcaat agtctcaggg gactacagag agggtgggaa gcccttaaat 7980
   atctgggaag tcttgtgcag tactggggtc aggagctaaa aaagagtact attagtctgg 8040
   ttgataccat agcaatagca gtagctgaag gaacagatag gattatagaa ttagtacaag 8100
   gactttgtag agctatctac agcataccta gaagaataag acagggcttt gaagcagctt 8160
   tgcaataaaa tggggggcaa gtggtcgaaa agtagcatag ttggatggcc tgctataagg 8220
   gagagaatga gaagaactga gccagcagca gatggggtgg gagcagtatc tcgagacctg 8280
   gaaaaacatg gagcaatcac gagtagcaat acagcagcta ctaatgagga ttgtgcctgg 8340
   ctggaagcac aagaggaggg ggaggtgggt tttccagtca gacctcaggt acctttaaga 8400
   ccaatgactt acaagggagc tgtagatctt agcttctttt taaaagaaaa ggggggactg 8460
   gaagggttaa tttactctaa gaaaaggcaa gagatccttg atttgtgggt ctatcacaca 8520
   caaggctact tccctgattg gcacaactac acaccaggac caggggtcag attcccactg 8580
   acttttgggt ggtgcttcaa gctagtacca gttgacccaa gggaagtaga agaggccaac 8640
   gagggagaag acaactgctt gctacaccct gtgtgccagc atggaatgga ggatgatcac 8700
   agagaagtat taaagtggaa gtttgacagt caactagcac acagacacag ggcccgcgaa 8760
   ctacatccgg agttttacaa agactgctga cacagaaggg actttccgcg gggactttcc 8820
   actggggcgt tctaggaggt gtggtctggc gggactggga gtggtcaacc ctcaaatgct 8880
   gcatataagc agctgctttt cgcctgtact gggtctctct agtcagacca gatctgagcc 8940
   tgggagctct ctggctaact agggaaccca ctgcttaagc ctcaataaag cttgccttga 9000
   ggggctagag cggccgccac cgcggtggag ctccagcttt tgttcccttt agtgagggtt 9060
   aattgcgcgc tggcgatc                                               9078
<210> 2
   <211> 4288
   <212> DNA
   <213> Human immunodeficiency virus
<400> 2
   gctaggtacc taatgggcgc cagggccagc atcctgaggg gcggcaagct ggacaagtgg 60
   gagaagatca ggctgaggcc cggcggcaag aagcactaca tgctgaagca cctggtgtgg 120
   gccagcaggg agctggagag gttcgccctg aaccccggcc tgctggagac cagcgagggc 180
   tgcaagcaga tcatgaagca gctgcagagc gccctgcaga ccggcaccga ggagctgagg 240
   agcctgttca acaccgtggc caccccctac tgcgtgcaca ccgagatcga cgtgagggac 300
   accagggagg ccctggacaa gatcgaggag gagcagaaca agatccagca gaagacccag 360
   caggccaagg aggccgacgg caaggtgagc cagaactacc ccatcgtgca gaacctgcag 420
   ggccagatgg tgcaccagcc catcagcccc aggaccctga atgcatgggt gaaggtggtg 480
   gaggagaagg ccttcagccc cgaggtgatc cccatgttca gcgccctgag cgagggcgcc 540
   accccccagg acctgaacac catgctgaac accgtgggcg gccaccaggc cgccatgcag 600
   atcctgaagg acaccatcaa cgaggaggcc gccgagtggg acaggctgca ccccgtgcac 660
   gccggcccca tcgcccccgg ccagatgagg gagcccaggg gcagcgacat cgccggcacc 720
   accagcaacc tgcaggagca gatcgcctgg atgaccagca acccccccgt gcccgtgggc 780
   gacatctaca agaggtggat catcctgggt ttaaacaaga tcgtgaggat gtacagcccc 840
   accagcatcc tggacatcaa gcagggcccc aaggagccct tcagggacta cgtcgacagg 900
   ttcttcaaga ccctgagggc ggagcaggcc acccagggcg tgaagaactg gatgaccgac 960
   accctgctgg tgcagaacgc caaccccgac tgcaagacca tcctgagggc cctgggcccc 1020
   ggcgccagca tcgaggagat gatgaccgcc tgccagggcg tgggcggccc cagccacaag 1080
   gccaaggtgc tggccgaggc catgagccag accaacagcg ccatcctgat gcagaggagc 1140
   aacttcaagg gcagcaagag gatcgtgaag tgcttcaact gcggcaagga gggccacatc 1200
   gccaggaact gcagggcccc caggaagaag ggctgctgga agtgcggcaa ggagggccac 1260
   cagatgaagg actgcaccga gaggcaggcc aacttcctgg gcaagatctg gcccagccac 1320
   aagggcggcc ccggcaactt cctgcagaac aggcccgagc ccaccgcccc ccccgaggag 1380
   agcttcaggt tcgaggagga gaccaccacc cccagccaga agcaggagcc catcgacaag 1440
   gagctgtacc ccctgaccag cctgaagagc ctgttcggca acgaccccag cagccaggaa 1500
   ttcttcaggg agaacctggc cctgccccag ggcagggcca gggagttcag cagcgagcag 1560
   accagggcca acagccccac caggggcgag ctgcaggtgt ggggcaggga caacaacagc 1620
   atcagcgagg ccggcgccaa caggcagggc accatcagct tcaacttccc ccagatcacc 1680
   ctgtggcaga ggcccctggt gaccatcaag atcggcggcc agctgaagga ggccctgctg 1740
   aacaccggcg ccggcgacac cgtgctggag gacctgaacc tgcccggcaa gtggaagccc 1800
   aagatgatcg gcggcatcgg cggcttcatc aaggtgaggc agtacgagca gatccccatc 1860
   gagatctgcg gccacaaggc catcggcacc gtgctggtgg gccccacccc cgtgaacatc 1920
   atcggcagga acctgctgac ccagctgggc tgcaccctga acttccccat cagccccatc 1980
   gagaccgtgc ccgtgaagct gaagcccggc atggacggcc ccaaggtgaa gcagtggccc 2040
   ctgaccgagg agaagatcaa ggccctgacc gccatctgcg acgagatgga gaaggagggc 2100
   aagatcacca agatcggccc cgagaacccc tacaacaccc ccatcttcgc catcaagaag 2160
   aaggacagca ccaagtggag gaagctggtg gacttcaggg agctgaacaa gaggacccag 2220
   gacttctggg aggtgcagct gggcatcccc caccccgccg gcctgaagaa gaagaagagc 2280
   gtgaccgtgc tggacgtggg cgacgcctac ttcagcatcc ccctgtacga ggacttcagg 2340
   aagtacaccg ccttcaccat ccccagcagg aacaacgaga cccccggcat cagctaccag 2400
   tacaacgtgc tgccccaggg ctggaagggc agcctggcca tcttccagag cagcatgacc 2460
   atcgaggagc tgatctacag caagaagagg caggagatcc tggacctgtg ggtgtaccac 2520
   acccagggct acttccccga ctggcacaac tacacccccg gccccggcgt gaggttcccc 2580
   ctgaccttcg gctggtgctt caagctggtg cccgtggacc ccagggaggt ggaggaggcc 2640
   aacgagggcg aggacaactg cctgctgcac cccgtgtgcc agcacggcat ggaggacgac 2700
   cacagggagg tgctgaagtg gaagttcgac agccagctgg cccacaggca cagggccagg 2760
   gagctgcacc ccgagttcta caaggactgc atgggcggca agtggagcaa gagcagcatc 2820
   gtgggctggc ccgccatcag ggagaggatg aggaggaccg agcccgccgc cgacggcgtg 2880
   ggcgccgtga gcagggacct ggagaagcac ggcgccatca ccagcagcaa caccgccgcc 2940
   accaacgagg actgcgcctg gctggaggcc caggaggagg gcgaggtggg cttccccgtg 3000
   aggccccagg tgcccctgag gcccatgacc tacaagggcg ccgtggacct gagcttcttc 3060
   ctgaaggaga agggcggcct ggagggcctg aggcagcacc tgctgaggtg gggcttcacc 3120
   acccccgaca agaagcacca gaaggagccc cccttcctgt ggatgggcta cgagctgcac 3180
   cccgacaagt ggaccgtgca gcccacccag ctgcccgaga aggacagctg gaccgtgaac 3240
   gacatccaga agctggtggg caagctgaac tgggccagcc agatctaccc cggcatcaag 3300
   gtgaggcagc tgtgcaagct gctgaggggc gccaaggccc tgaccgacat cgtgcccctg 3360
   accgaggagg ccgagctgga gctggccgag aacagggaga tcctgaagga gcccgtgcac 3420
   ggcgtgtact acgaccccag caaggacctg atcgccgaga tccagaagca gggccaggag 3480
   cagtggacct accagatcta ccaggagccc ttcaagaacc tgaagaccgg caagtacgcc 3540
   aagatgagga ccgcccacac caacgacgtg aagcagctga ccgaggccgt gcagaagatc 3600
   gccatggagg gcatcgtgat ctggggcaag acccccaagt tcaggctgcc catccagaag 3660
   gagacctggg agacctggtg gaccgactac tggcaggcca cctggatccc cgagtgggag 3720
   ttcgtgaaca cccctcccct ggtgaagctg tggtatcagc tggagaagga ccccatcgtg 3780
   ggcgtggaga ccttctacgt ggacggcgcc gccaacaggg agaccaagat cggcaaggcc 3840
   ggctacgtga ccgacagggg caggaagaag atcgtgagcc tgaccgagac caccaaccag 3900
   aagaccgagc tgcaggccat ctgcatcgcc ctgcaggaca gcggcagcga ggtgaacatc 3960
   gtgaccgaca gccagtacgc cctgggcatc atccaggccc agcccgacaa gagcgagagc 4020
   gagctggtga accagatcat cgagcagctg atgaagaagg agagggtgta cctgagctgg 4080
   gtgcccgccc acaagggcat cggcggcaac gagcaggtgg acaagctggt gagcagcggc 4140
   atcaggaagg tgctgaagac cctggagccc ttcaggaagc agaaccccgg catcgtgatc 4200
   taccagtaca tggacgacct gtacgtgggc agcgacctgg agatcggcca gcacaggacc 4260
   aagtaaagat ctctcgagga gctcaagc 4288
<210> 3
   <211> 2605
   <212> DNA
   <213> Human immunodeficiency virus
<400> 3
   gcggcgggta ccgaattcgc cgccagcatg gacagggcca agctgctgct gctgctgctg 60
   ctgctgctgc tgccccaggc ccaggccgtg ggcaacctgt gggtgaccgt gtactacggc 120
   gtgcccgtgt ggaagggcgc caccaccacc ctgttctgcg ccagcgacgc caaggcctac 180
   gacaccgagg tgcacaacgt gtgggccacc cacgcctgcg tgcccgccga ccccaacccc 240
   caggagatgg tgctggagaa cgtgaccgag aacttcaaca tgtggaagaa cgagatggtg 300
   aaccagatgc aggaggacgt catcagcctg tgggaccaga gcctgaagcc ctgcgtgaag 360
   ctgacccccc tgtgcgtgac cctggagtgc aggaacgtga gcagcaacag caacgacacc 420
   taccacgaga cctaccacga gagcatgaag gagatgaaga actgcagctt caacgccacc 480
   accgtggtga gggacaggaa gcagaccgtg tacgccctgt tctacaggct ggacatcgtg 540
   cccctgacca agaagaacta cagcgagaac agcagcgagt actacaggct gatcaactgc 600
   aacaccagcg ccatcaccca ggcctgcccc aaggtgacct tcgaccccat ccccatccac 660
   tactgcaccc ccgccggcta cgccatcctg aagtgcaacg acaagatctt caacggcacc 720
   ggcccctgcc acaacgtgag caccgtgcag tgcacccacg gcatcaagcc cgtggtgagc 780
   acccagctgc tgctgaacgg cagcctggcc gagggcgaga tcatcatcag gagcgagaac 840
   ctgaccaaca acgtgaaaac catcatcgtg cacctgaacc agagcgtgga gatcgtgtgc 900
   accaggcccg gcaacaacac caggaagagc atcaggatcg gccccggcca gaccttctac 960
   gccaccggcg acatcatcgg cgacatcagg caggcccact gcaacatcag cgaggacaag 1020
   tggaacgaga ccctgcagag ggtgagcaag aagcttgccg agcacttcca gaacaagacc 1080
   atcaagttcg ccagcagcag cggcggcgac ctggaggtga ccacccacag cttcaactgc 1140
   aggggcgagt tcttctactg caacaccagc ggcctgttca acggcgccta cacccccaac 1200
   ggcaccaaga gcaacagcag cagcatcatc accatcccct gcaggatcaa gcagatcatc 1260
   aacatgtggc aggaggtggg cagggccatg tacgcccctc ccatcaaggg caacatcacc 1320
   tgcaagagca acatcaccgg cctgctgctg gtgagggacg gcggcaccga gcccaacgac 1380
   accgagacct tcaggcccgg cggcggcgac atgaggaaca actggaggag cgagctgtac 1440
   aagtacaagg tggtggagat caagcccctg ggcgtggccc ccaccaccac caagaggagg 1500
   gtggtggaga gggagaagag ggccgtgggc atcggcgccg tgttcctggg cttcctgggc 1560
   gtggccggca gcaccatggg cgccgccagc atcaccctga ccgtgcaggc caggcagctg 1620
   ctgagcggca tcgtgcagca gcagagcaac ctgctgaggg ccatcgaggc ccagcagcac 1680
   ctgctgcagc tgaccgtgtg gggcatcaag cagctgcaga ccagggtgct ggccatcgag 1740
   aggtacctga aggaccagca gctgctgggc atctggggct gcagcggcaa gctgatctgc 1800
   accaccgccg tgccctggaa cagcagctgg agcaacaaga gccagaagga gatctgggac 1860
   aacatgacct ggatgcagtg ggacaaggag atcagcaact acaccaacac cgtgtacagg 1920
   ctgctggagg agagccagaa ccagcaggag aggaacgaga aggacctgct ggccctggac 1980
   agctggaaga acctgtggag ctggttcgac atcaccaact ggctgtggta catcaagatc 2040
   ttcatcatca tcgtgggcgg cctgatcggc ctgaggatca tcttcgccgt gctgagcatc 2100
   gtgaacaggg tgaggcaggg ctacagcccc ctgagcttcc agaccctgac ccccaacccc 2160
   ggcggccccg acaggctggg caggatcgag gaggagggcg gcaagcagga cagggacagg 2220
   agcatcaggc tggtgaacgg cttcctggcc ctggcctggg acgacctgag gaacctgtgc 2280
   ctgttcagct accacaggct gagggacttc accctggtgg ccgccagggt ggtggagctg 2340
   ctgggcagga acagcctgag gggcctgcag aggggctggg aggccctgaa gtacctgggc 2400
   agcctggtgc agtactgggg ccaggagctg aagaagagca ccatcagcct ggtggacacc 2460
   atcgccatcg ccgtggccga gggcaccgac aggatcatcg agctggtgca gggcctgtgc 2520
   agggccatct acagcatccc caggaggatc aggcagggct tcgaggccgc cctgcagtga 2580
   taaagatctc tcgaggagct caagc                                      2605

## Beschreibung

Die vorliegende Erfindung betrifft ein Polynukleotid, wie es im anhängenden Anspruch 1 definiert ist. Die vorliegende Erfindung betrifft ferner Polypeptide, wie sie im anhängenden Anspruch 8 definiert sind. Die Polynukleotide und Polypeptide können als Arzneimittel, Impfstoffe oder Diagnostika, insbesondere für die Behandlung, Prävention und Diagnose von HIV-Infektionen, verwendet werden.

In Anbetracht des Ausmaßes und der globalen Verbreitung der durch das humane Immundefizienz Virus (HIV) verursachten Pandemie mit einer, bis zum Ende dieses Jahrhunderts, geschätzten Anzahl von weltweit mehr als 40 Millionen Infizierten (davon mehr als 90% in Entwicklungsländern) stellt die Entwicklung einer wirksamen HIV-Vakzine eine der größten Herausforderungen an die moderne industrialisierte Welt dar. Bislang wird die Entwicklung eines erfolgreichen HIV-Impfstoffs jedoch noch immer durch die komplizierte Biologie des Virus sowie seine komplexe Interaktion mit dem Immunsystem des Wirtes limitiert. Die wenigen Impfstoff- Kandidaten, die bis zum heutigen Zeitpunkt in Entwicklungsländern in Phase 3-Studien ausgetestet wurden, basierten hauptsächlich auf den externen Gykoproteinen gp120 oder gp160 von HIV Typ-1. Der Ausgang der Studien war jedoch eher enttäuschend: Die Impfstoffe waren nicht nur nicht in der Lage, breit kreuz-neutralisierende Antikörper- und T-Zell-Reaktionen hervorzurufen. Sie konnten nicht einmal Infektionsdurchbrüche verhindern, die bei einigen Impflingen beobachtet worden sind. Einer der Gründe für dieses Versagen liegt sicherlich in den extensiven Sequenzvariationen zwischen den verwendeten Antigenen, welche von laboradaptierten Virusstämmen abstammten, und den genetisch divergenten Viren, welche in den Testregionen (z.B. Thailand) kursierten.

Phylogenetische Analysen der weltweit zirkulierenden HIV-Stämme haben eine Hauptgruppe (M) mit 10 verschiedenen Sequenz-Subtypen (A-J) (Kostrikis *et al.* 1995; Leitner und Albert, 1995; Gaywee *et al.* 1996; World Health Organisation Network for HIV Isolation and Characterization, 1994), die im Hüllprotein Sequenzvariationen von bis zu 24 % aufweisen, und außerdem die Viren der O-Gruppe identifiziert, die sich in einigen Leserahmen um mehr als 40 % von den Viren der M-Gruppe unterscheiden (Loussert Ajaka *et al.* 1995; Myers *et al.* 1996; Sharp *et al.* 1995; Sharp *et al.* 1999). Zudem entwickelt sich HIV durch die rasche Anhäufung von Mutationen und Intersubtyp-Rekombinationen immer weiter. Unterschiedliche Subtypen, welche innerhalb der Population einer geographischen Region kozirkulieren, stellen die molekulare Grundlage für die Erzeugung und Ausbreitung von gruppenübergreifenden Intersubtyp-Mosaikviren dar. Obwohl die weltweit verbreiteten HIV-1-Varianten durch Serologie und Heteroduplex-DNA-Analysen intensiv untersucht wurden, beruhen die meisten phylogenetischen Analysen auf Sequenzen des Hüllproteins, da für viele der prävalenten Subtypen und eine Vielzahl von rekombinanten Formen keine vollsequenzierten Genome vorliegen.

Für die überwiegende Mehrheit der weltweit neuen HIV-1-Infektionen sind Viren des Subtyps Non-B (also *Nicht*-B-Varianten) verantwortlich. Den Viren des Subtyps C fällt dabei im Hinblick auf die Gesamtzahl von Infizierten sowie der weiten Verbreitung von Neu-Infektionen, insbesondere in Süd-Amerika und Asien, eine herausragende Rolle zu. Auf Grund dessen hat die Charakterisierung von Viren des Subtyps C eine herausragende Priorität für diagnostische, therapeutische oder präventive Zwecke.

Mit Ausnahme von Thailand lagen bis vor kurzem nur begrenzte Informationen über die Verteilung und molekulare Charakteristik von in Asien vorkommenden HIV-1-Stämmen vor. Nach Schätzungen der WHO breitet sich HIV am schnellsten in Süd- und Südost-Asien aus, welche schon bald die weltweit größte Region mit HIV-Epidemie sein wird. China unterliegt ähnlichen sozialen und ökonomischen Strukturen und unterhält zu diesen Regionen unmittelbare ethnische und wirtschaftliche Verbindungen. In vielen Provinzen Chinas konnte seit Anfang 1995 ein rasanter Anstieg von HIV-Infektionen beobachtet werden. Verglichen mit allen von 1985 bis 1994 dokumentierten 1774 Fällen an HIV und AIDS, wurden im Jahr 1995 alleine schon 1421 Fälle und im Jahr 1997 mehr als 4000 Fälle nachgewiesen. Die WHO geht von mehr als 400.000 HIV-Infektionen in China bis Ende 1997 aus, mit bis dahin 6400 Todesfällen und einer geschätzten Anzahl von 4000 Todesfällen allein im Jahre 1997. Im kürzlich veröffentlichten nationalen HIV-Molekularepidemiologischen Bericht wurde gefunden, daß die Thai-Stämme des Prototyp-Subtyps B und des Subtyps B' in Yunnan, einer Provinz im Südwesten von China, die angrenzt an das Drogendreieck von Myanmar, Laos und Thailand (Graf et al. 1998), durch Benutzer von Drogen und durch Sammelstellen für kontaminiertes Blut und Plasma bis nach Zentral- und Ost-China verbreitet wurden. In den frühen 90er Jahren wurde dann in die gleiche Region eine zweite Epidemie eingeschleppt, sehr wahrscheinlich durch mit Stämmen des Subtyps C infizierte indische IDUs (intravenous drug user), also Menschen aus Indien, die Drogen intravenös verwenden (Luo *et al.* 1995; Shao *et al.* 1999). Innerhalb weniger Jahre verbreiteten sich die Viren des Subtyps C durch Drogenschmuggel schnell in Süd-, Zentral- und sogar in Nordwest-China und verursachten eine weitere Verbreitung der Epidemie innerhalb Chinas. Einem kürzlich veröffentlichten nationalen HIV-Molekularepidemiologischen Untersuchungsbericht zufolge sind fast alle mit Viren des Subtyps C infizierten Personen IDUs und machen damit etwa 40% aller HIV-infizierten IDUs in China aus. Das legt nahe, daß die Viren des Subtyps C zu den wichtigsten Subtypen von HIV-1 zählen, die unter IDUs in China prävalent sind (Shao et al. 1998, Shao et al. 1994).

Dies legt nahe, daß sich die HIV-Epidemie unter den IDUs in China innerhalb weniger Jahre von einem einzelnen vorherrschenden Subtyp (B) auf mindestens 2 vorherrschende Subtypen, B-Thai und C, ausgeweitet hat, was die Möglichkeit der Intersubtyp-Rekombination erhöht. Nach unserem bisherigen Kenntnisstand über Variabilität und Antigenizität unterschiedlicher Virus-Stämme sollten Diagnostika, Therapeutika und Impfstoffe auf regio-nale Virus-Stämme angepasst sein. Die Anzahl molekularer Reagenzien für Viren des *Nicht*-B-Subtyps sind jedoch noch extrem limitiert. Außer für Viren des Subtyps B oder C sind bislang nur wenige nicht-rekombinante molekulare Klone und wenige Mosaikgenome verfügbar. Was HI-1-Viren des Subtyps C betrifft, sind bislang nur nicht-rekombinante Vertreter und vier A/C-Rekombinanten publiziert, die alle aus Afrika, Süd-Amerika oder Indien stammen (Luo *et al.* 1995; Gao *et al.* 1998; Lole *et al.* 1999). Darüber hinaus beschränken sich die bislang gesammelten Daten über Viren des Subtyps C in China auf genetische Subtypisierungen des env-Gens (Luo *et al.* 1995; Yu *et al.* 1997; Salminen *et al.* 1995).

Mehrere klinische Studien zur Bekämpfung von HIV-Infektionen wurden bislang mit Vakzinen durchgeführt. Die enttäuschenden Ergebnisse, die bei klinischen Studien beobachtet wurden, beinhalten wiederholt berichtete Infektionsdurchbrüche bei den Impflingen. Dies wurde vor allem auf die umfangreichen Sequenzvariationen zwischen den verabreichten Hüllproteinen und dem infektiösen Input-Virus zurückgeführt, was tatsächlich vorwiegend auf eine unzureichende Charakterisierung der in einer bestimmten geographischen Region zirkulierenden Viruspopulation zurückzuführen ist. Dies resultierte in der Erzeugung von humoralen und - in geringerem Ausmaß - von zellvermittelten Immunantworten gegen virale Antigene, welche nicht relevant waren für die in der Population des Testgebietes zirkulierenden Viren. Zudem konnte gezeigt werden, daß gering affine, spezifisch gegen das Hüllprotein gerichtete Antikörper nicht nur keine neutralisisierenden Eigenschaften besitzen, sondern darüberhinaus sogar zu einer Verstärkung der Infektion mittels Komplement- oder Fc-Rezeptor beitragen. Desweiteren erwiesen sich die ausgewählten Antigene und Verabreichungssysteme als extrem schwach für die Induktion der zellvermittelten Immunantwort.

Angesichts eines Mangels an genauer Kenntnis über Subtyp-übergreifende protektive Immunantworten sowie über die komplexe Situation in Entwicklungsländern, wo bekannter-maßen viele Subtypen von HIV-1 kozirkulieren, sollten Impfstoff-Präparationen Mischungen von repräsentativen Antigenen enthalten. Somit besteht also ein Bedarf an der Isolierung und Charakterisierung von Viren des Subtyps C, insbesondere für die Klonierung der kodierenden Region.

Die Aufgabe der vorliegenden Erfindung wird durch den in den Patentansprüchen definierten Gegenstand gelöst.

Die nachfolgenden Figuren erläutern die vorliegende Erfindung.

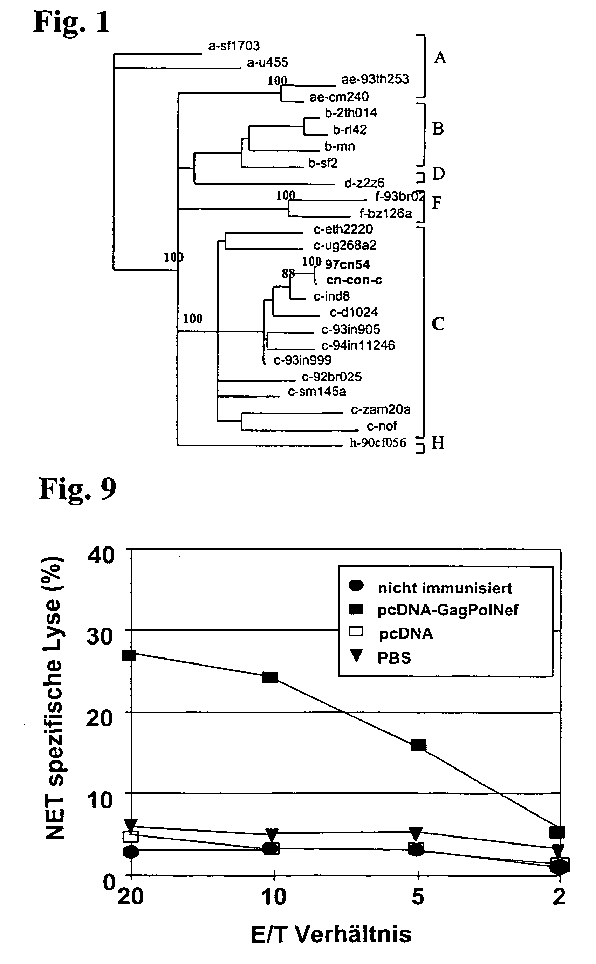

Figur 1 zeigt eine Darstellung der phylogenetischen Verwandtschaft der das env-Gen C2V3 kodierenden Region des Klons 97cn54 zu den Vertretern der wichtigen Subtypen von HIV-1 (M-Gruppe). "cn-con-c" steht für die env-Konsensussequenz der HIV-1-Stänmme des Sub-typs C, welche in China prävalent sind. Der phylogenetische Stammbaum wurde mittels der *"neighbour joining*"-Methode erstellt. Die Werte an den Knoten geben die "*bootstraps*" in % an, welche die Eingruppierung rechts unterstützt. Nur "*bootstrap*"-Werte, die 70% erreichen oder überschreiten, sind angegeben. Die Klammern rechts stellen die Sequenzen der wichtigsten Subtypen von HIV-1, M-Gruppe, dar.

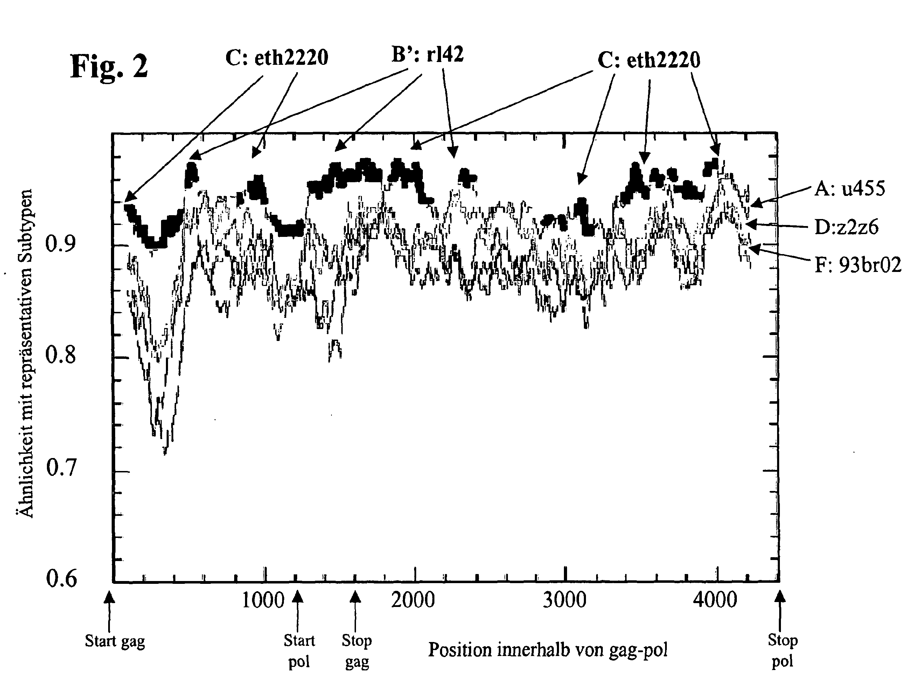

Figur 2 zeigt eine Darstellung der RIP-Analyse (Recombinant Identification Program), Version 1.3, des gesamten für gagpol kodierenden Bereichs von 97cn54 (Fenstergröße: 200, Schwellenwert für die statistische Signifikanz: 90%, Umgang mit Lücken: STRIP). Die Positionen der offenen Leserahmen von gag und pol sind durch Pfeile im Diagramm oben dargestellt. Die RIP-Analyse basierte auf Hintergrundvergleichen unter Verwendung von Referenzsequenzen, die von ausgewählten Virus-Stämmen abstammten, die die wichtigsten Subtypen von HIV-1 darstellen. Standardvertreter sind durch verschiedene Farben markiert, wie angezeigt. Die x-Achse gibt die Nukleotid-Positionen entlang des Sequenzvergleichs an. Die y-Achse gibt die Ähnlichkeit von 97cn54 mit den aufgelisteten Referenz-Subtypen an.

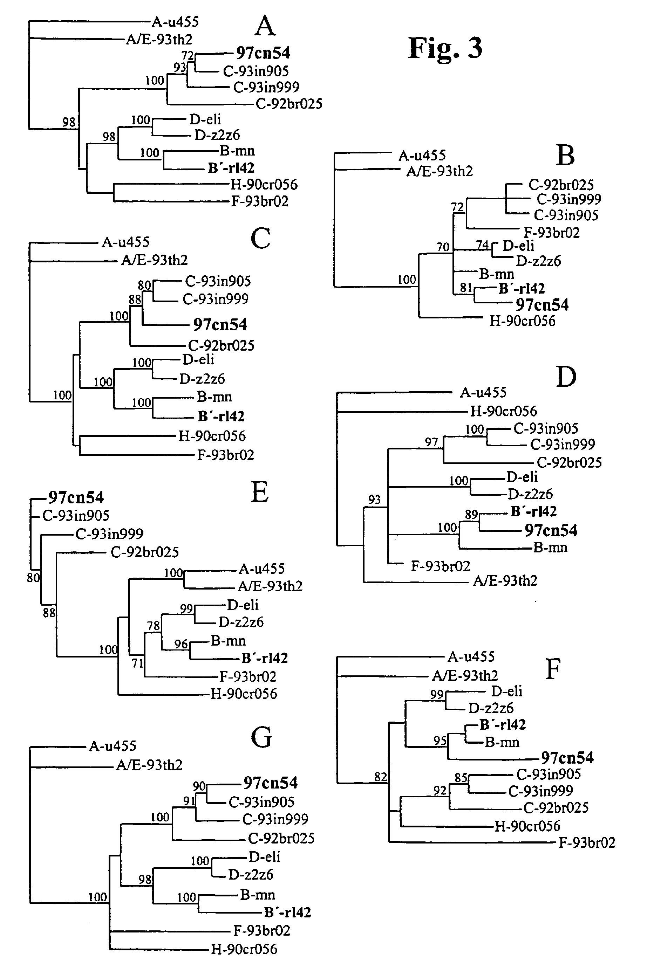

Figur 3 zeigt eine Darstellung der phylogenetischen Verwandtschaft verschiedener Regionen innerhalb der von 97cn54 abgeleiteten Leserahmen von gagpol mit Standard-Vertretern der wichtigsten Subtypen von HIV-1 (M-Gruppe). Unter Verwendung der *"neighbour joining"-*Methode basierend auf den folgenden Sequenzabschnitten: (A) Nukleotide 1-478, (B) 479-620, (C) 621-1290, (D) 1291-1830, (E) 1831-2220, (F) 2221-2520 und (G) 2521-2971 wurden phylogenetische Stammbäume erstellt. Die angegebenen Positionen beziehen sich auf das erste Nukleotid des offenen Leserahmens von gag. Graue Bereiche kennzeichnen Cluster der analysierten Sequenzen entweder mit von Subtyp C (A, C, E, G) oder von Subtyp B (B, D, F) abgeleiteten Referenzstämmen. Die Werte an den Knoten geben die "*bootstrap*"-Werte in Prozent an, durch die das Cluster rechts bestätigt wurde. Es werden nur "*bootstrap*"-Werte von 70% oder mehr gezeigt.

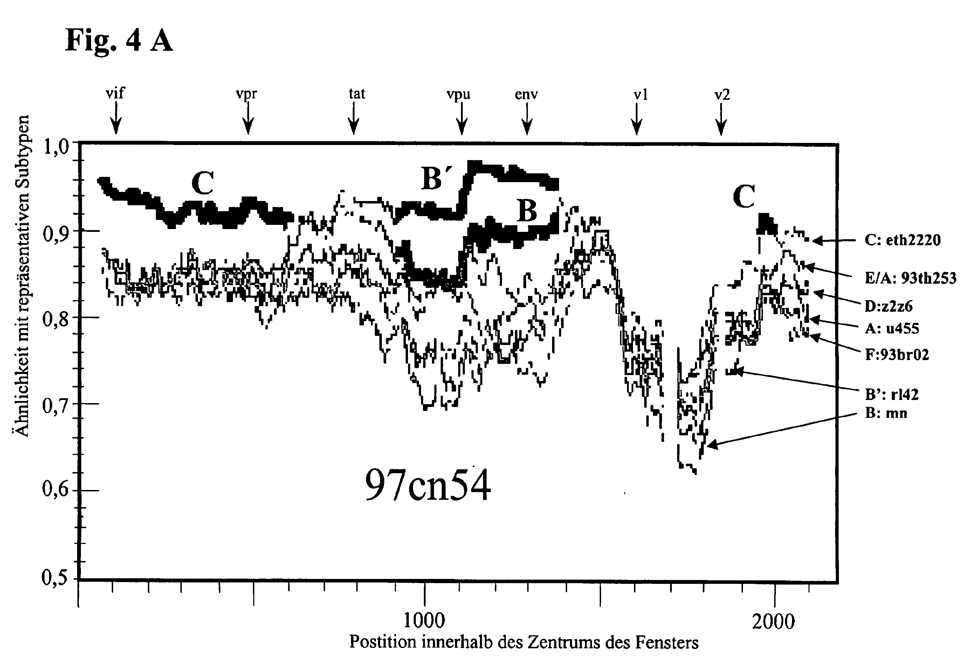

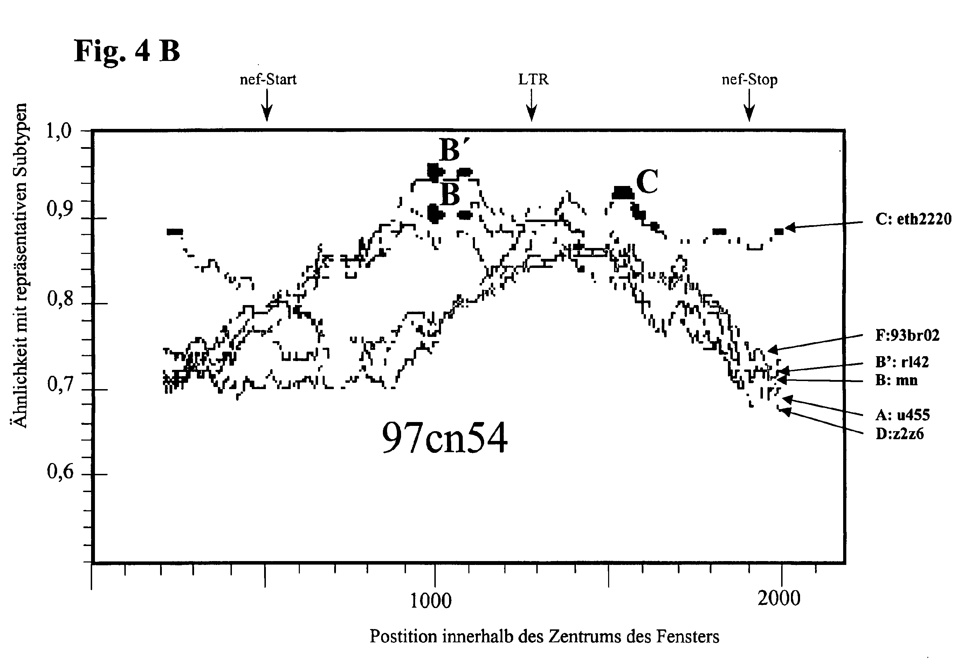

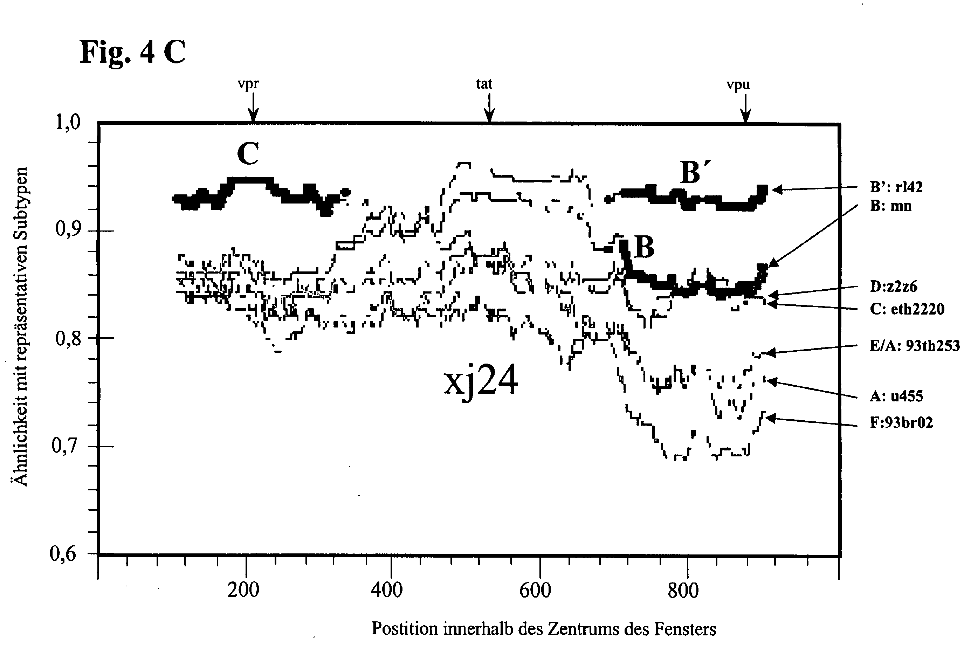

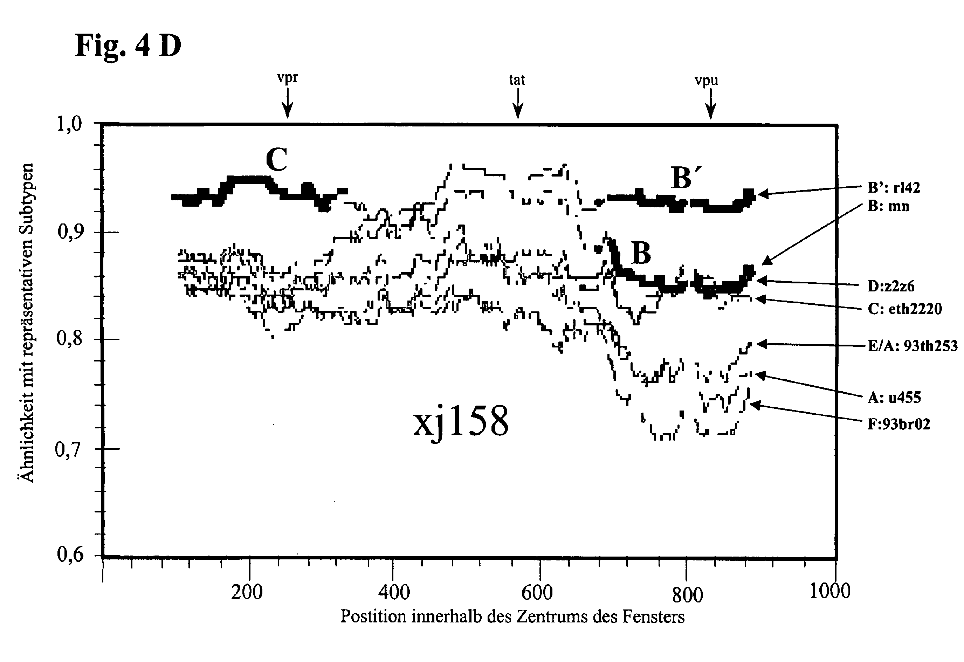

Figur 4 zeigt eine Darstellung der RIP-Analyse, Version 1.3, von verschiedenen Regionen von 97cn54 (Fenstergröße: 200, Schwellenwert für die statistische Signifikanz: 90%, Umgang mit Lücken: STRIP). Die Analyse umfaßte (A) einen Sequenzbereich von 1500 bp Länge vom Startkodon des vif-Gens bis zum 5'-Ende von env einschließlich vif, vpr, dem ersten Exon von tat und rev, vpu und den ersten 200 bp des env-Gens und (B) ein etwa 700 bp langes Fragment, das 300 bp vom 3'-Ende von env, die das komplette nef-Gen und Teile der 3'-LTR-Region umfassen, überlappt. Die Positionen der Startkodons vpr, tat, vpu, env, nef und das 5'-Ende der 3'-LTR-Region sind jeweils oben in den Diagrammen durch Pfeile gekennzeichnet. Die RIP-Analyse basierte auf Hintergrund-Vergleichen unter Verwendung von Sequenzen, die von ausgewählten Virusstämmen abgeleitet waren, die die wichtigsten Subtypen von HIV-1 repräsentierten. Die angegebenen Standardvertreter sind durch verschiedene Farben gekennzeichnet. Die x-Achse gibt die Nukleotidpositionen entlang des Sequenzvergleichs an. Die y-Achse gibt die Ähnlichkeit von 97cn54 mit den aufgelisteten Referenz-Subtypen an. (C) und (D) zeigen RIP-Analysen von Sequenzen von zwei unabhängigen C-Isolaten (xj24 und xj158) aus China, die das vpr- und vpu-Gen einschließlich des ersten Exons von tat überlappen.

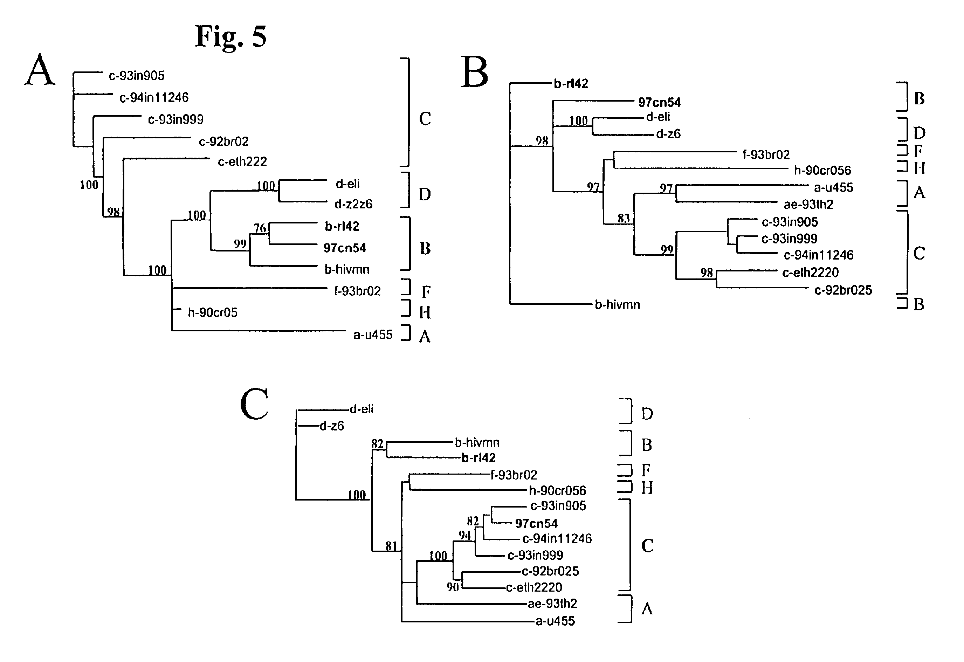

Figur 5 zeigt die Analyse eines phylogenetischen Stammbaums. Phylogenetische Stammbäume wurden unter Verwendung der *'heighbour joining"-Methode* erstellt basierend auf (A) einem 380 bp langen Fragment, das 150 bp vom 3'-Ende des vpr-Gens bis zum Ende des vpu-Leserahmens überlappt, (B) den ersten 290 bp der kodierenden Region von nef und (C) auf den 320 bp am 3'-Ende des nef-Gens. Die Werte an den Knoten geben die "*bootstrap*"-Werte in Prozent an, durch die das Cluster rechts bestätigt wurde. Es werden nur "*bootstrap*"-Werte von 70% oder mehr gezeigt. Die Klammern rechts stellen die wichtigsten Subtyp-Sequenzen von HIV-1, Gruppe M, dar.

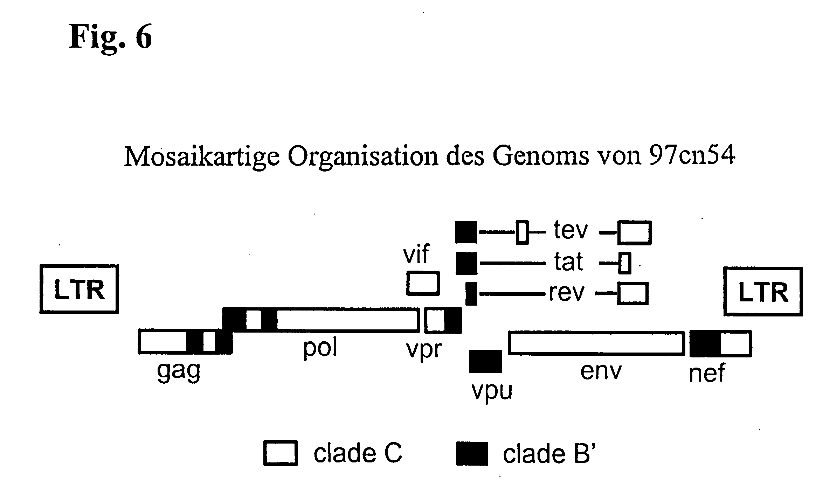

Figur 6 ist eine schematische Darstellung der mosaikartigen Organisation des Genoms von 97cn54.

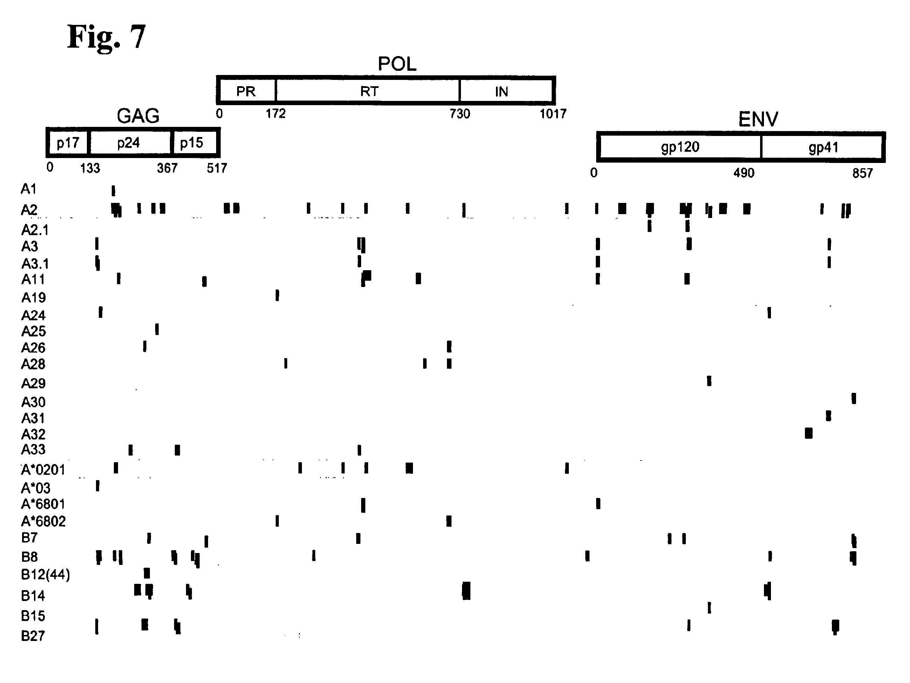

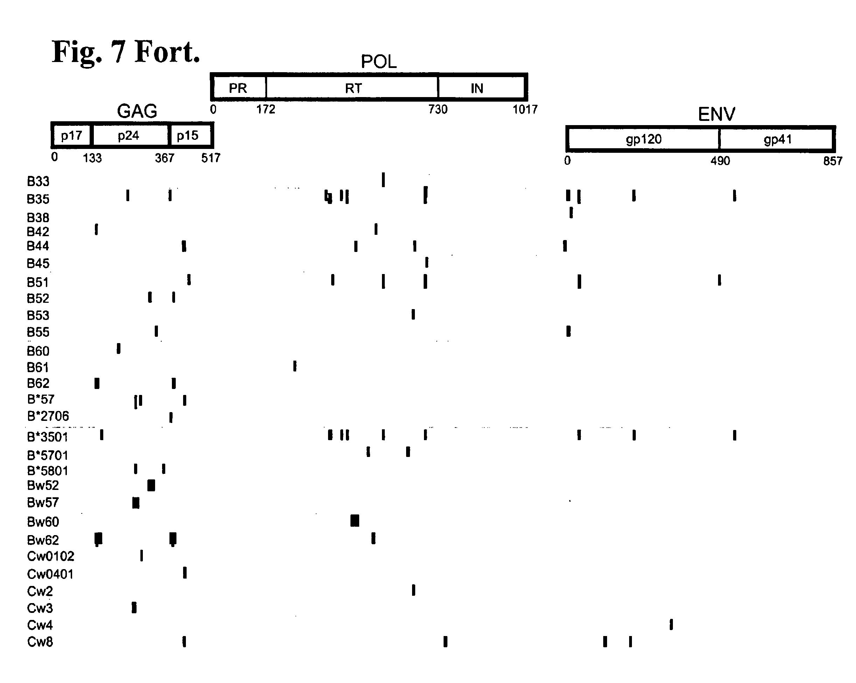

Figur 7 ist eine Darstellung des Vergleichs zwischen bekannten und experimentell nachgewiesenen CTL-Epitopen des Prototyps B (HIV-1_{LAI}) und den entsprechenden Aminosäure-Sequenzen der Polypeptide gag, pol und env des Stammes 97cn54 vom Subtyp C. Die funktionellen Domänen in GAG (p17 Matrix, p24 Kapsid, p15 Nukleokapsid und Linker-Protein), POL (PR Protease, RT Reverse Transkriptase, IN Integrase) und ENV (gp 120 äußeres Glykoprotein, gp41 Transmembranprotein) sind entsprechend bezeichnet. Die Zahlen unterhalb der offenen Leserahmen geben die Aminosäure- Position relativ zu den aminoterminalen Enden der Polypeptide an. Haplotyp-Restriktionen der bekannten CTL-Epitope von HIV-1_{LAI} sind am linken bzw. rechten Rand angegeben. Die grünen Balken kennzeichnen Sequenz-Identität zwischen dem bekannten Epitop und der ensprechenden Sequenz vom Subtyp C, blaue Balken bedeuten 2 oder weniger konservative Fehlpaarungen. Rote Balken stellen vom Subtyp C abgeleitete Sequenz-Bereiche mit mehr als 2 konservativen Fehlpaarungen oder nicht-konservative Substitutionen im Vergleich zu dem entsprechenden von LAI abgeleiteten Epitop dar.

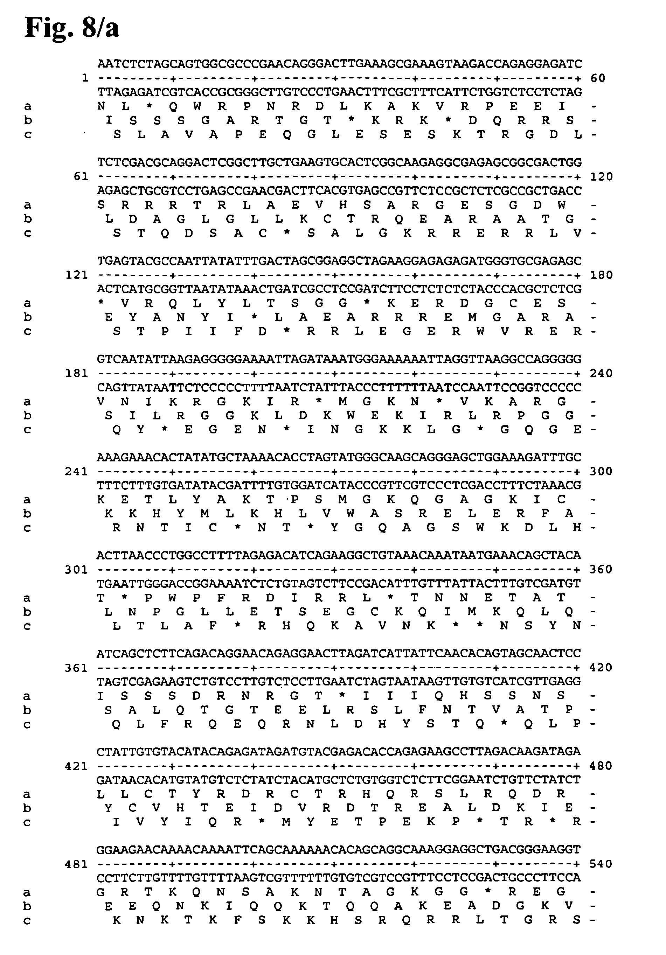

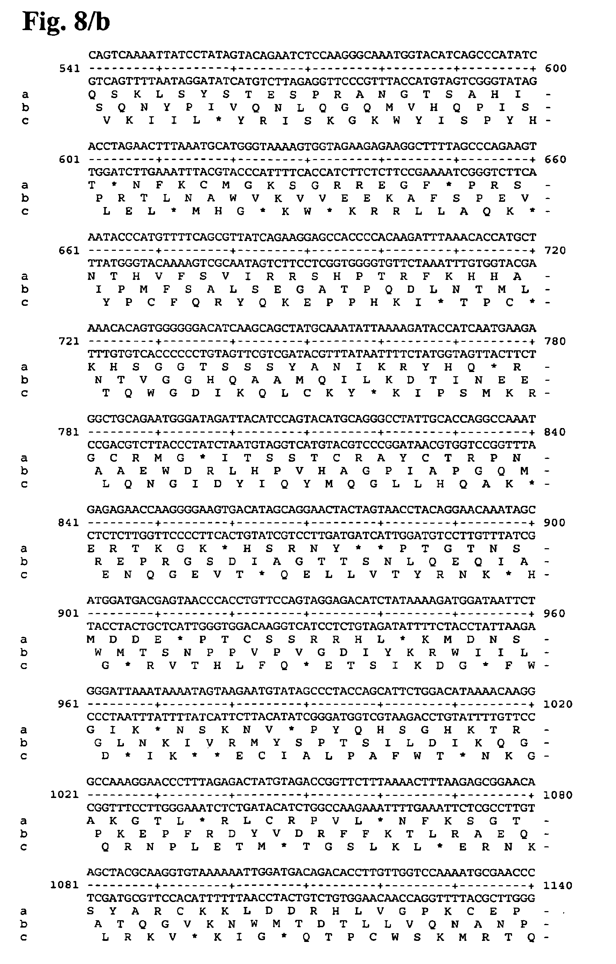

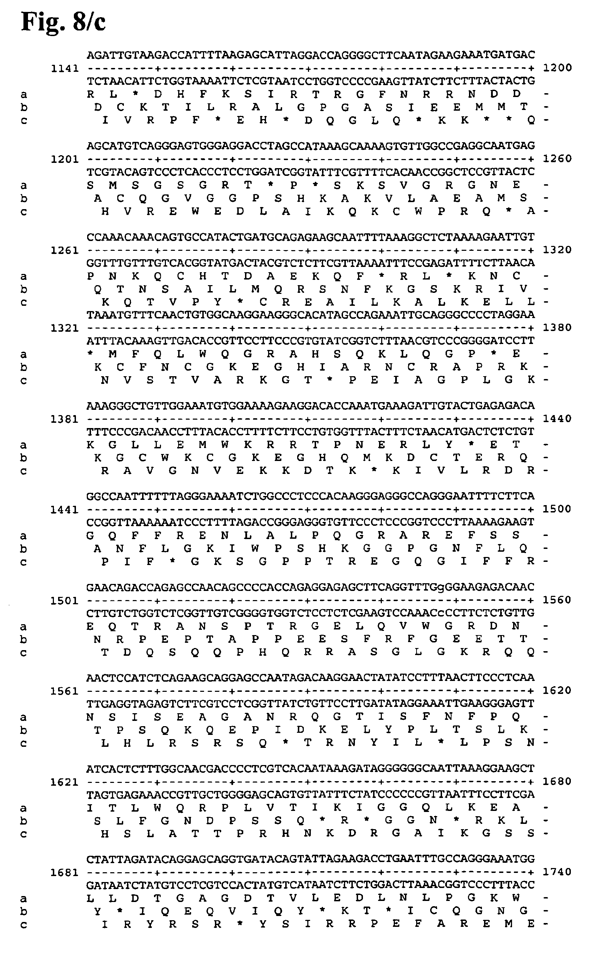

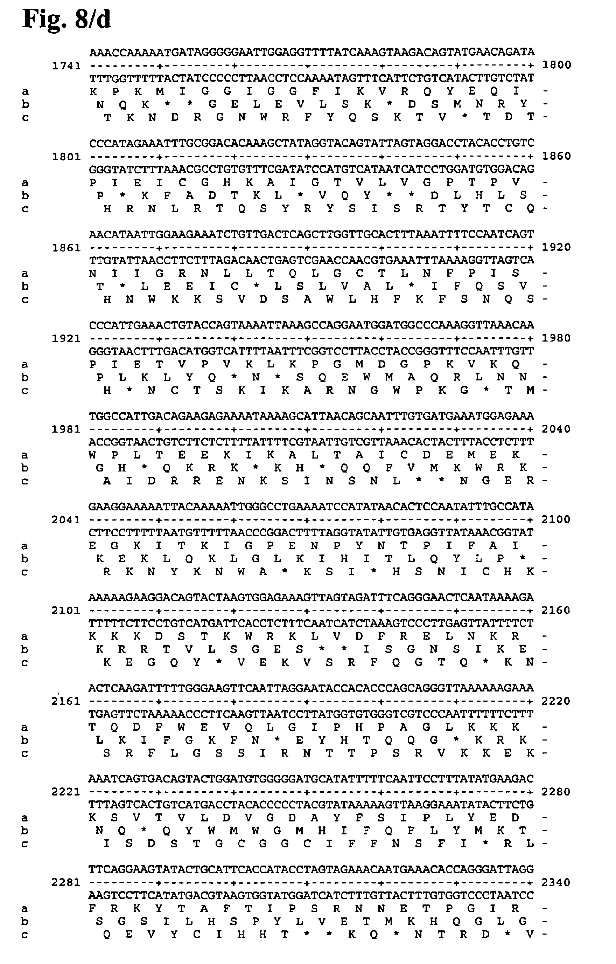

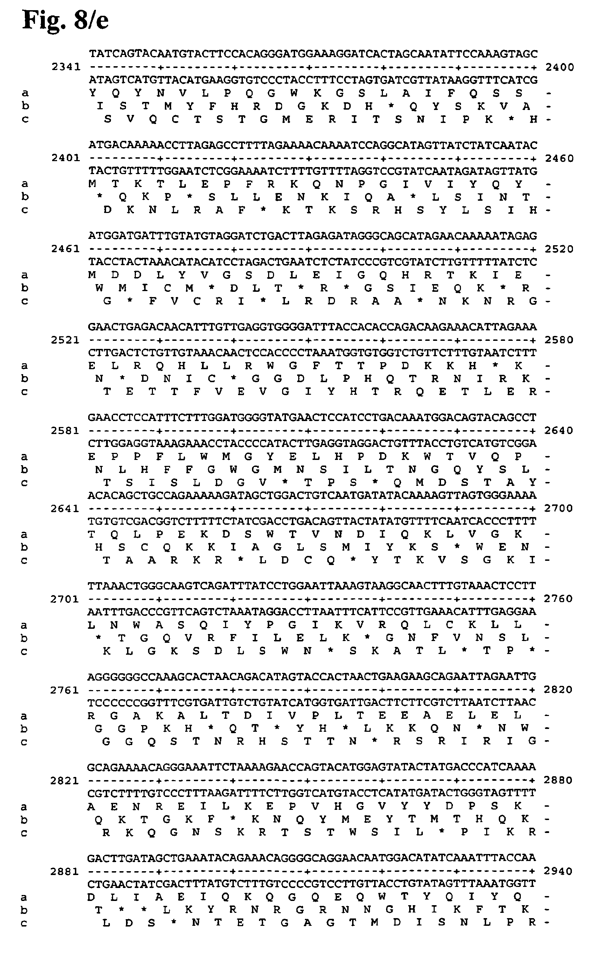

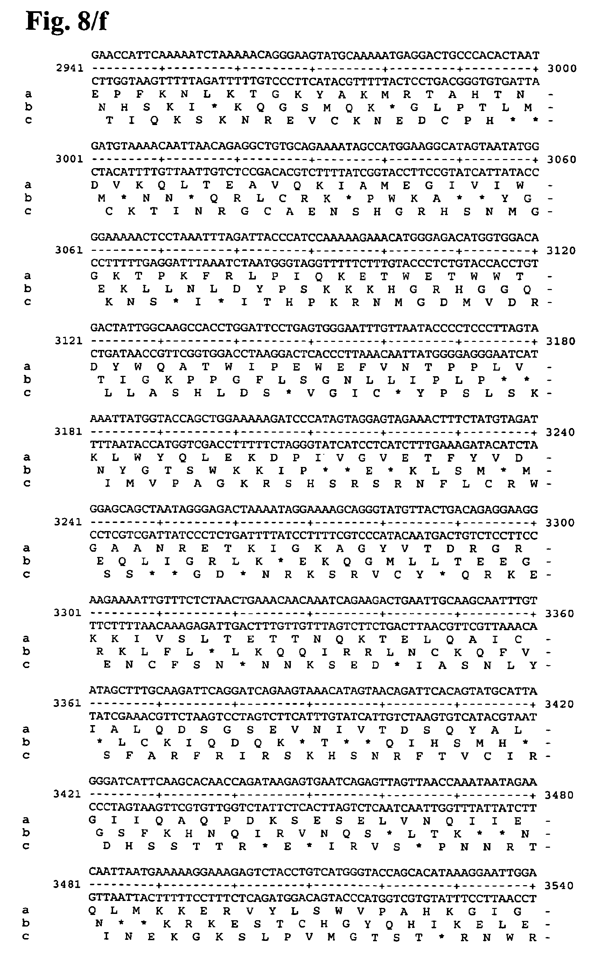

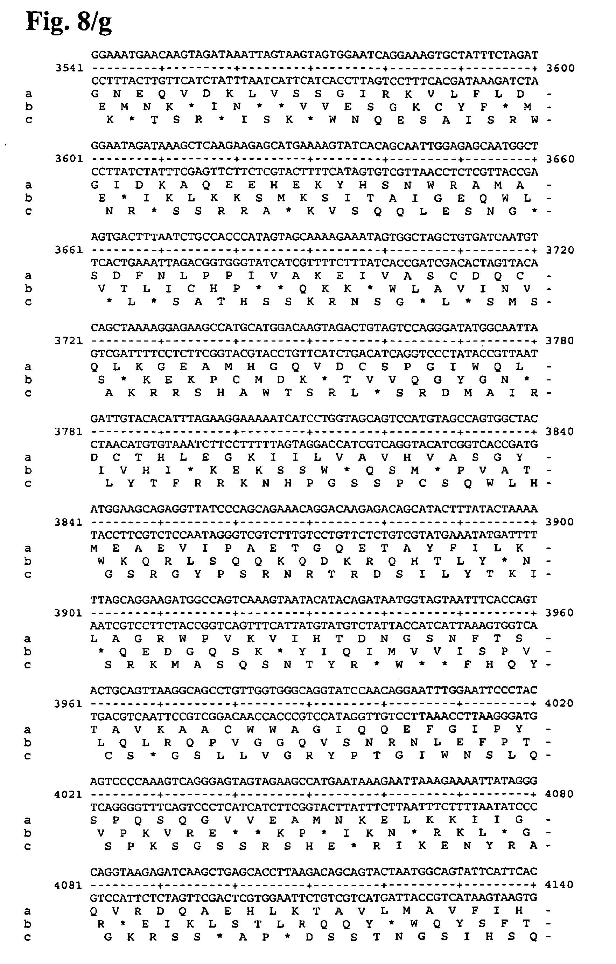

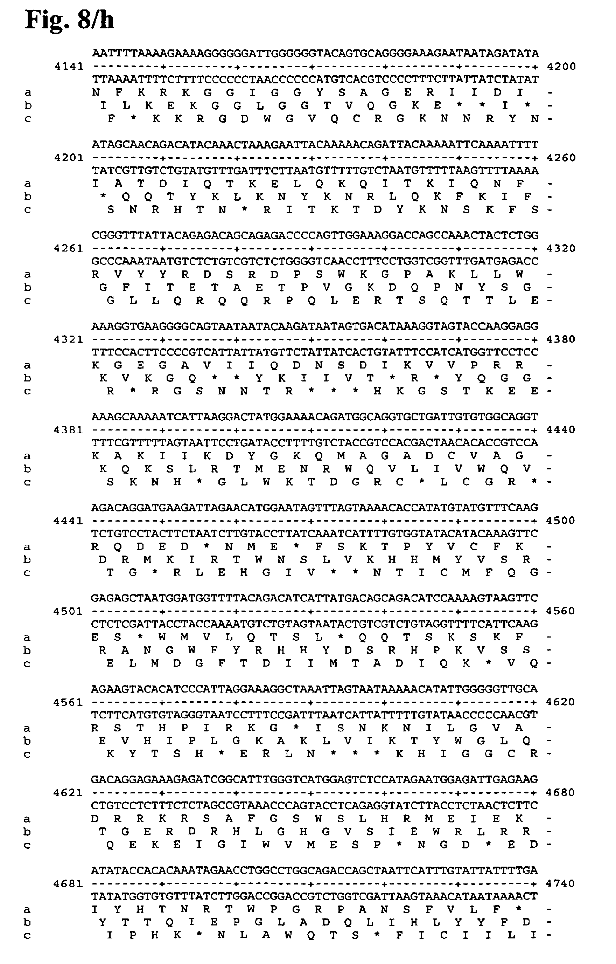

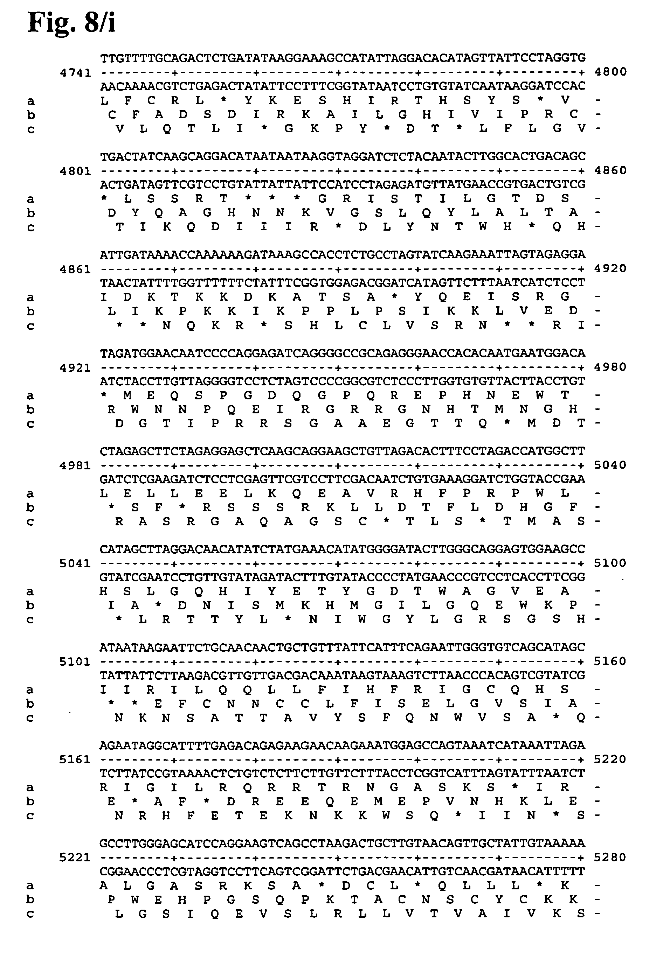

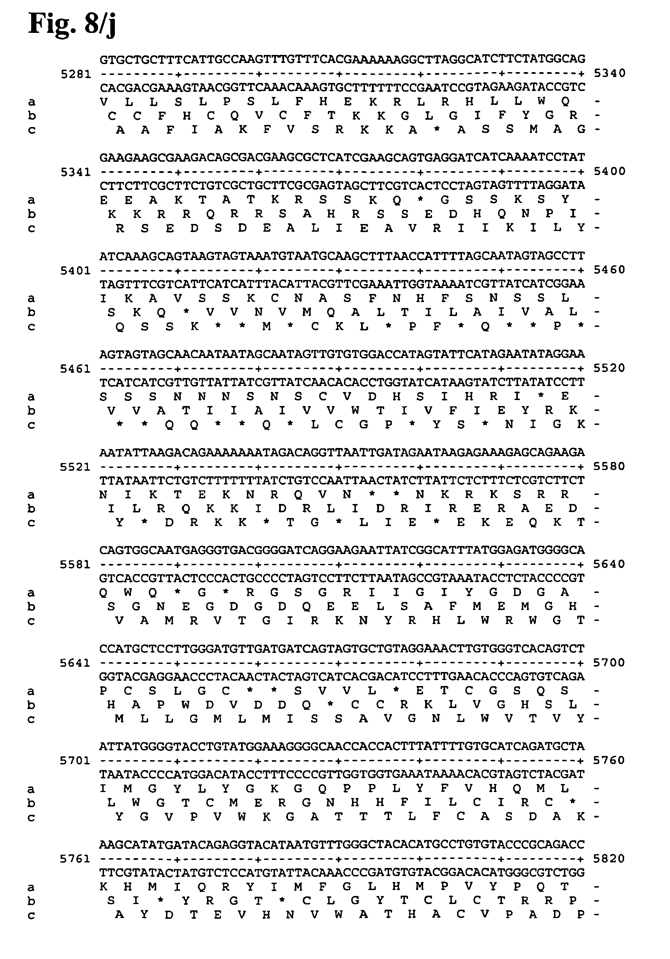

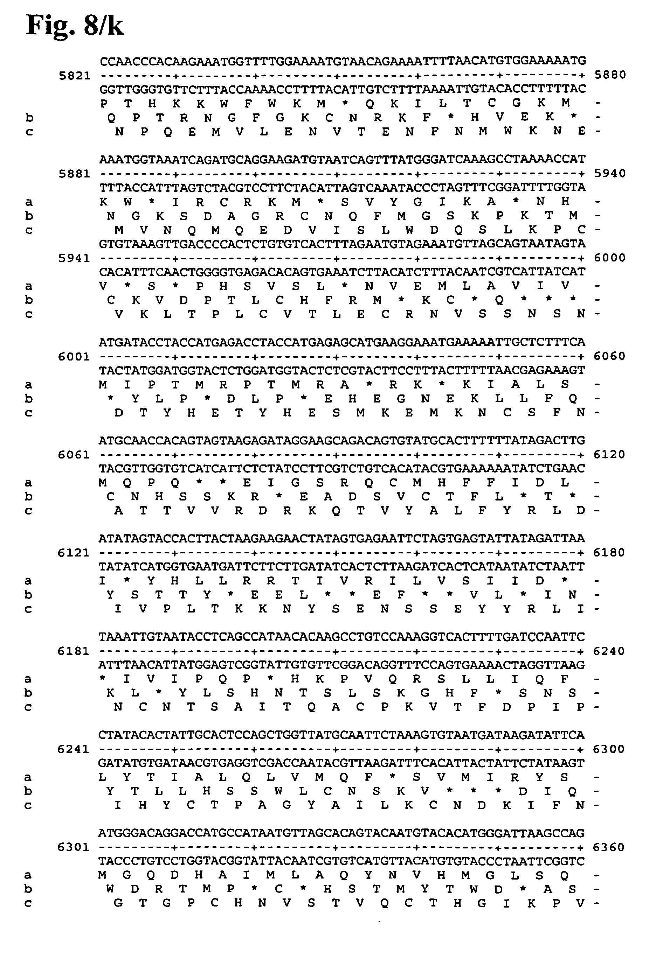

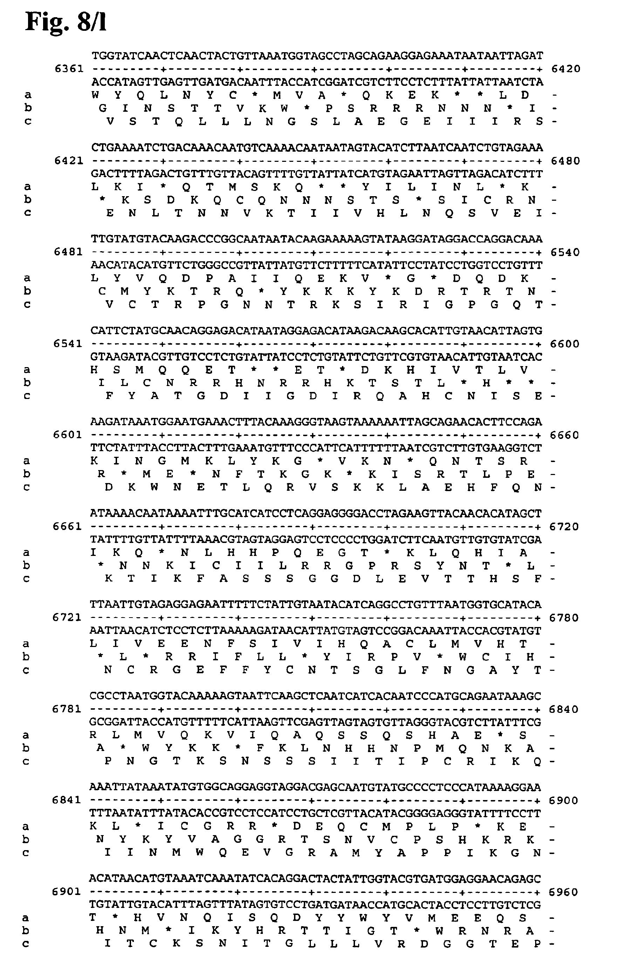

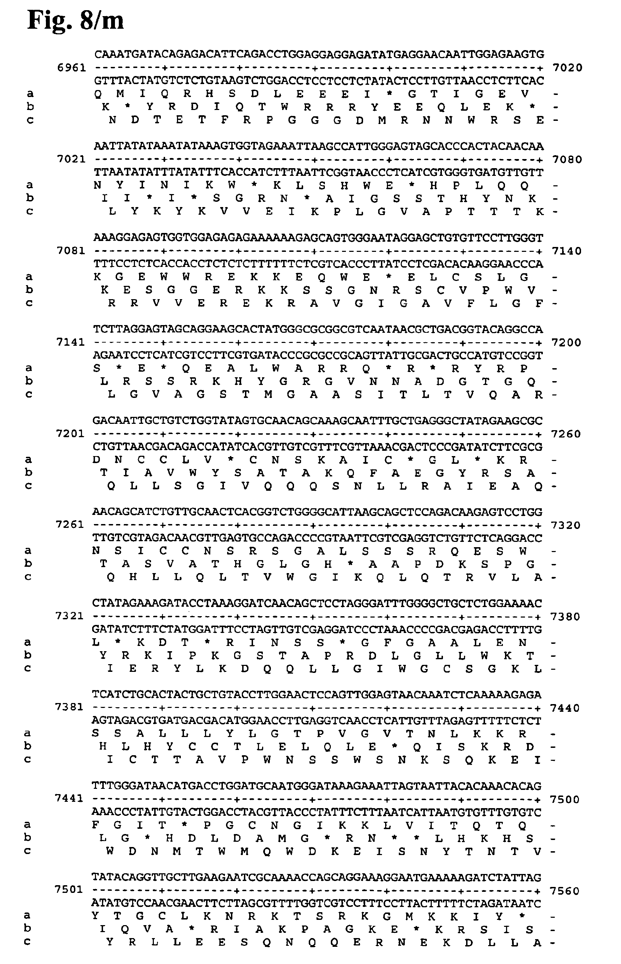

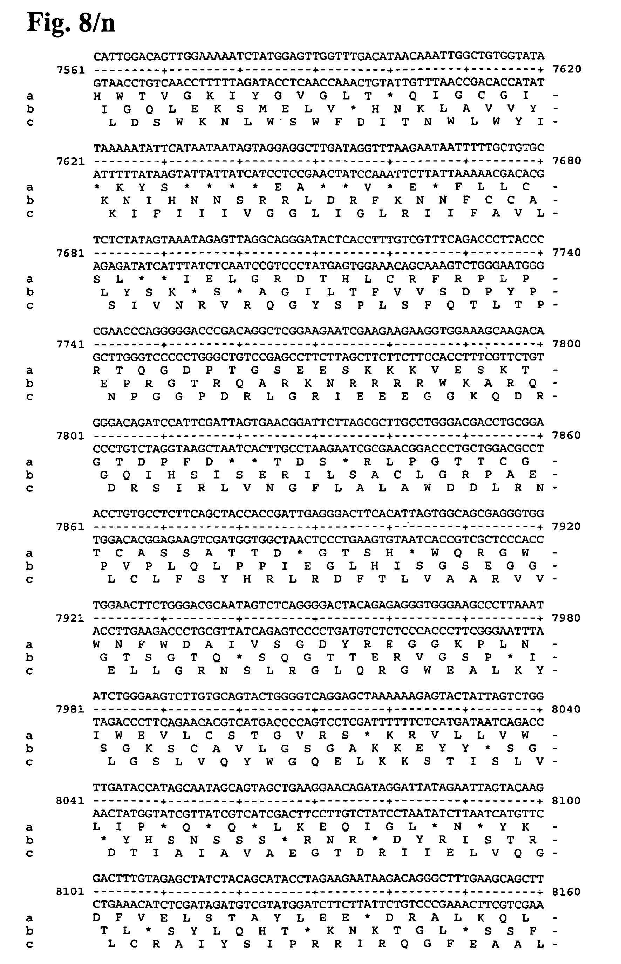

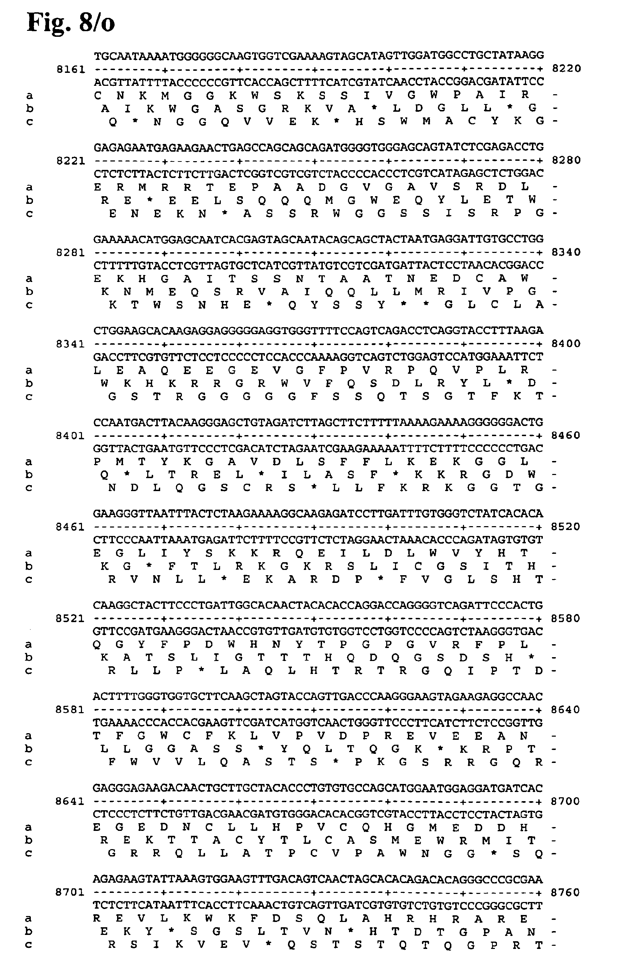

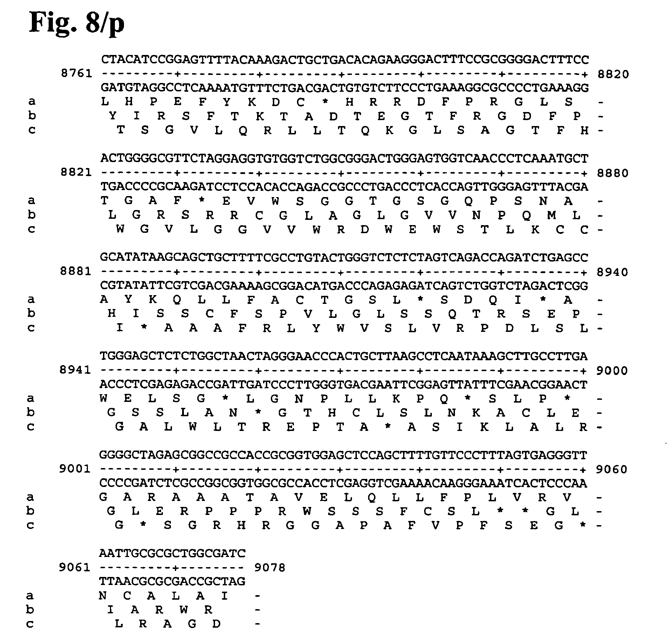

Figur 8 zeigt die vollständige kodierende Nukleotidsequenz von 97cn54 von HIV-1, Subtyp C (SEQ ID NO:1), mit den entsprechenden Aminosäuren im Einbuchstaben-Kode. Alle 3 Leserahmen sind angegeben. Die Sternchen stellen Stopp-Kodons dar.

Figur 9 zeigt in einer Darstellung das Ergebnis der Aktivitäten zytotoxischer T-Zellen in Milzzellen von BALB/c Mäusen nach intramuskulärer Immunisierung mit den angezeigten DNA-Plasmiden. Lymphoide Zellen, gewonnen 3 Wochen nach der Primärimmunisierung aus jeweils 5 Mäusen pro Gruppe, wurden mit AMQMLKETI (Einbuchstabencode) Gag-Peptid beladenen syngenen P815 Mastozytom Zellen (bestrahlt mit 20,000 rad) kokultiviert. Kontrollen schlossen Milzzellen nicht-immunisierter Mäuse, stimuliert mit Peptid-beladenen P815 Zellen ein. Zytotoxische Effektor-Zellpopulationen wurden nach einer 5-tägigen Kultur *in vitro* geerntet. Die zytotoxischen Antworten wurden gegen A20 Zellen, beladen mit dem oben aufgeführte nonameren Peptid oder gegen unbeladene A20 Zellen, in einem Standard ⁵¹Cr Freisetzungstest ausgelesen. Die gezeigten Daten repräsentieren die Mittelwerte aus jeweils Dreifachansätzen. Die ermittelten Standardabweichungen lagen jeweils unter 15% gemessen am Mittelwert.

Die Begriffe "Epitop" oder "Antigene Determinante", wie nachfolgend verwendet, bedeuten eine immunologisch determinante Gruppe eines Antigens, das spezifisch von einem Antikörper erkannt wird. Ein Epitop kann Aminosäuren in räumlicher oder diskontinuierlicher Konformation umfassen und umfaßt mindestens 3, vorzugsweise mindestens 5, Aminosäuren. Ein Epitop kann auch ein einzelnes Segment einer Polypeptid-Kette umfassend eine kontinuierliche Aminosäure-Sequenz umfassen.

Der Begriff "Polynukleotid", wie nachfolgend verwendet, bezieht sich auf ein einzel- oder doppelsträngiges Heteropolymer aus Nukleotid-Einheiten beliebiger Länge, wobei diese entweder Ribo- oder Desoxyribonukleotide sein können. Der Begriff umfaßt auch modifizierte Nukleotide.

Der Begriff "Derivat", wie nachfolgend verwendet, bezeichnet eine Nukleinsäure, die ebenfalls das oder die Polypeptide kodiert, die von einer anderen Nukleotidsequenz kodiert werden, obwohl sich ihre Nukleotidsequenz von der anderen Nukleotidsequenz unterscheidet. In diesem Sinne bezeichnet der Ausdruck "Derivat" auch Äquivalente der anderen Nukleotidsequenz, die aufgrund der Degeneration des genetischen Codes vorliegen. Unter den Begriff Derivat fallen z.B. Nukleinsäuren, die die gleichen Polypeptide wie die Nukleotidsequenz gemäß SEQ ID NO:1, 2 oder 3 kodieren, aber eine andere Nukleotidsequenz aufweisen, oder es fallen ferner Nukleinsäure-Fragmente unter den Begriff, die das gleiche Polypeptid kodieren wie Nukleinsäure-Fragmente der Nukleotidsequenz gemäß SEQ ID NO: 1, 2 oder 3.

Der Begriff "Polypeptid", wie nachfolgend verwendet, bezieht sich auf eine Kette von mindestens 2 Aminosäure-Resten, die durch Peptidbindungen miteinander verbunden sind. Der Begriff umfaßt daher alle Aminosäure-Ketten, z.B. Oligopeptide und Proteine. Der Begriff bezieht sich auch auf solche Aminosäure-Ketten, bei denen eine oder mehrere Aminosäure(n) modifiziert ist(sind), z.B. durch Acetylierung, Glykosylierung oder Phosphorylierung.

Der Begriff "kontinuierliche Sequenz" und "Fragmente", wie nachfolgend verwendet, bezieht sich auf einen linearen Abschnitt von Nukleotiden oder Aminosäuren, der von einer Referenz-Sequenz stammt, z.B. von den Sequenzen der vorliegenden Erfindung, wie sie in dem Sequenzprotokoll wiedergegeben sind.

Der Begriff "selektive Hybridisierung" bzw. "selektiv hybridisierbar", wie nachfolgend verwendet, bezieht sich auf Hybridisierungsbedingungen, bei denen zwei Polynukleotide unter stringenten Hybridisierungsbedingungen Duplex-Nukleotidmoleküle bilden. Diese Bedingungen sind im Stand der Technik bekannt und z.B. in Sambrook et al., Molecular Cloning, Cold Spring Harbour Laboratory (1989), ISBN 0-87969-309-6 beschrieben. Beispiele für stringente Hybridisierungsbedingungen sind: (1) Hybridisierung in 4 x SSC bei 65°C oder (2) Hybridisierung in 50% Formamid in 4 x SSC bei 42°C, jeweils gefolgt von mehreren Waschschritten in 0,1 x SSC bei 65°C (1 Stunde lang).

Der Begriff "viraler Vektor " oder "bakterieller Vektor", wie nachfolgend verwendet, bezieht sich auf gentechnisch veränderte Viren oder Bakterien, mit denen sich die in den SEQ ID NO:1, 2 oder 3 ausgeführten DNA-Sequenzen, davon abgeleitete Derivate, Fragmente, Sequenzen kodierend für Epitope oder Epitop-Strings in unterschiedliche Zellen, bevorzugt in antigenpräsentierende Zellen wie beispielsweise Dendritische Zellen einbringen lassen. Ein bakterieller Vektor kann darüber hinaus geeignet sein, ein von SEQ ID NO:1, 2 oder 3 kodiertes Polypeptid, davon abgeleitete Epitope oder Epitop-Strings direkt zu exprimieren.

Ein Aspekt der vorliegenden Erfindung betrifft eine Nukleotidsequenz, wie sie in SEQ ID NO: 1, SEQ ID NO: 2 oder SEQ ID NO: 3 beschrieben ist. Zunächst wurde eine molekulare Epidemiologie-Studie unter mehr als 100 IDUs aus China, die seropositiv bezüglich des Subtyps C von HIV-1 waren, durchgeführt, um notwendige Informationen zu sammeln über repräsentative virale Genome von im wesentlichen voller Länge. Die Genotypisierung auf der Basis der konstanten Region 2 und der variablen Region 3 (C2V3) innerhalb des Gens für das virale Hüll-Glykoprotein offenbarte die höchste Homologie der am meisten prävalenten Virusstämme, die in ganz China zirkulieren, zu Sequenzen des Subtyps C indischen Ursprungs. Basierend auf diesen Ergebnissen wurde aus peripheren mononukleären Blutzellen (PBMC) von einem ausgewählten HIV-infizierten IDU direkt ein Genom von im wesentlichen voller Länge amplifiziert und subkloniert, das die am meisten prävalente Klasse der C-Stämme, die in ganz China zirkulieren, darstellt. Die Sequenzanalyse identifizierte eine Mosaikstruktur, was auf extensive Intersubtyp-Rekombinationsvorgänge zwischen den Genomen der prävalenten C- und (B')-Subtyp-Thai-Virusstämme jener geographischen Region deutet. Eine RIP-Analyse (Recombinand Identification Program Analysis) und phylogenetisches "bootstrapping" legten insgesamt 10 Bruchstellen (i) in der für gagpol kodierenden Region, (ii) in vpr und am 3'-Ende des vpu-Gens und (iii) im offenem Leserahmen von nef nahe. Thai (B')-Sequenzen umfassen daher (i) mehrere Insertionen in der kodierenden Region von gagpol (Nukleotide 478-620, 1290-1830, 2221-2520, jeweils bezogen auf das erste Nukleotid innerhalb des Startkodon des Gag- bzw. des GagPol-Leserahmens), (ii) 3'-vpr, das komplette vpu, die ersten Exons von tat und rev (etwa 1000 Nukleotide beginnend etwa an Nukleotid 138 bezogen auf das Startkodon des Vpr-Leserahmens) und (iii) die 5'-Hälfte des nef-Gens (Nukleotide 1-300). Die übrigen Bereiche innerhalb der 9078 Nukleotide umfassenden Sequenz (SEQ ID NO: 1; Tabelle 3) weisen höchste Homologien zu bekannten Subtyp C Isolaten auf. Bruchstellen von 97cn54, die in der kodierenden Region von vpr/vpu bzw. im nef-Gen lokalisiert sind, wurden bei vielen Stämmen des Subtyps C, die von IDUs isoliert wurden, die in verschiedenen Gebieten Chinas leben, an ähnlichen Positionen gefunden. Dies legt eine gemeinsame Abstammung für die C/B'-rekombinanten Stämme nahe. Bei mehr als 50% der gut definierten CTL-Epitope, die vom Subtyp B abstammen, innerhalb von Gag und Pol und bei 10% der bekannten Epitope in Env, wurde gefunden, daß die Sequenzen innerhalb dieser C/B'-chimären Referenzstämme exakt übereinstimmen. Diese Ergebnisse können die Anstrengungen in bezug auf Impfstoffe in China deutlich erleichtern, indem außerordentlich wichtige Matritzen für die Konzeption von Impfstoffen bereitgestellt und Reagentien für die am besten geeigneten immunologischen/virologischen Ausleseverfahren entwickelt werden.

Die Verwendung der beschriebenen Sequenz gemäß vorliegender Erfindung, einer Sequenz von HIV-1, das den am meisten prävalenten C-Typ Virusstamm innerhalb Chinas darstellt, als Grundlage und Ausgangsmaterial ist für die Entwicklung von präventiv oder therapeutisch einsetzbaren Impfstoffen von Vorteil. Die notwendigen Konsequenzen für die Entwicklung eines erfolgreichen HIV-Impfstoffkandidaten sind (i) ein detailliertes Wissen über die jeweilige epidemiologische Situation und (ii) die Verfügbarkeit einer klonierten kodierenden Sequenz, die innerhalb einer geographischen Region oder einer bestimmten Bevölkerung den am meisten prävalenten Virusstamm repräsentiert. Solche Sequenzen stellen die Grundlage dar (i) für die rationale Konzeption von präventiv und therapeutisch einsetzbaren HN-Impfstoffkandidaten, (ii) für Entwicklung spezifischer Therapeutika, wie beispielsweise therapeutisch wirksamer Decoy-Oligonukleotiden und Proteine, Antisense-Konstrukte, Ribozyme und transdominant negativ wirksamer Mutanten (iii) für die Entwicklung lentiviraler Vektoren für die Gentherapie und (iv) die Herstellung von Reagenzien, die für Diagnose und Verlaufskontrolle der HIV-Infektion sowie die immunologische/virale Überwachung des Impfungsprozesses eingesetzt werden können.

Dies ist insbesondere zutreffend für Impfstoffkandidaten, die auf den HIV-Hüllproteinen beruhen, von denen gezeigt wurde, daß sie unter allen HIV-Proteinen die größte Variabilität aufweisen. Darüber hinaus wird ein erfolgreicher Impfstoff sehr wahrscheinlich beide Arme des Immunsystems induzieren müssen: neutralisierende Antikörper, idealerweise gerichtet gegen Konformations-Epitope im Hüllprotein sowie zellvermittelte Immunantworten (CD4-positive T-Helfer-Zellen, CD8-positive zytolytische T-Zellen, Zytokine vom Typ Th-1, β-Chemokine), erzeugt gegen Epitope verschiedener viraler Proteine. Das Konformations-Epitop gemäß der vorliegenden Erfindung besteht aus mindestens 3 Aminosäuren, vorzugsweise aus 5 oder mehr Aminosäuren, die bei der Antikörper-Bindung involviert sind. Konfoimationelle Epitope können sich auch aus mehreren Abschnitten entweder eines einzigen Proteins, oder - im Falle oligomerer Komplexe wie z.B. des trimeren Hüllglykoprotein-Komplexes - aus mehreren Abschnitten unterschiedlicher Untereinheiten zusammensetzen. Ein lineares Epitop gemäß der vorliegenden Erfindung variiert normalerweise in der Länge und umfaßt mindestens 8 Aminosäuren bis etwa 15 Aminosäuren oder mehr, wobei eine Länge von 9 bis 11 Aminosäuren insbesondere im Falle MHC Klasse I restringierter CTL-Epitope bevorzugt ist.

Die vorliegende Erfindung betrifft somit ferner Polypeptide, kodiert von der Nukleotidsequenz oder Fragment oder Derivat der Nukleotidsequenz gemäß SEQ ID NO:1, 2 oder 3, wie im Anspruch 8 definiert. Offenbart sind ferner Polypeptide, umfassend eine kontinuierliche Sequenz von mindestens 8 Aminosäuren, die von der Nukleotidsequenz oder Fragmenten oder Derivaten der Nukleotidsequenz gemäß SEQ ID NO:1, 2 oder 3 kodiert werden. Vorzugsweise umfasst das erfindungsgemäße Polypeptid eine antigene Determinante, die natürlicherweise in Infizierten eine Immunreaktion auslöst. Besonders bevorzugt sind Polypeptide, umfassend eine Aminosäuresequenz, kodiert von der Nukleotidsequenz gemäß SEQ ID NO:2 oder 3 oder dessen Derivate und Fragmente. Insbesondere bevorzugt sind Epitope umfassend einen kontinuierlichen Bereich von 9 bis 11 Aminosäuren, die identisch sind zwischen den durch SEQ ID NO:1 kodierten Polypeptiden und einem HIV-1_{LAI} Referenzisolat, oder die 2 oder weniger konservierte Aminosäuresubstitutionen innerhalb der 9 bis 11 Aminsäuren umfassenden Sequenz aufweisen. Beispiele für derartige Epitope sind in Beispiel 11 aufgeführt. Die erfindungsgemäßen Polypeptide können z.B. als Impfstoffe und Therapeutika oder zur Diagnostik verwendet werden.

Ein weiterer Aspekt der vorliegenden Erfindung betrifft ein Polynukleotid gemäß SEQ ID NO:1, 2 oder 3. Ferner offenbart ist ein Polynukleotid-Fragment der Nukleotidsequenz gemäß SEQ ID NO:1, 2 oder 3, oder ein Polynukleotid, das mindestens eine kontinuierliche Sequenz von Nukleotiden umfaßt, die zur selektiven Hybridisierung an die Nukleotidsequenz, wie sie in SEQ ID NO:1, 2 oder 3 dargestellt ist, in der Lage ist. Ferner offenbart sind Derivate der erfindungsgemäßen Polynukleotide oder Polynukleotid-Fragmente. Vorzugsweise umfasst das Polynukleotid oder das Polynukleotid-Fragment eine kontinuierliche Sequenz von mindestens 9 Nukleotiden, bevorzugterweise von mindestens 15 Nukleotiden, noch bevorzugterweise von mindestens 27 Nukleotiden, oder eine längere Sequenz. Das Polynukleotid oder das Polynukleotid-Fragment kann auch die kodierende Region der einzelnen HIV-Gene umfassen, wie z.B. von gag, pol, env. Beispiele sind in SEQ ID NO: 2 und SEQ ID NO: 3 angegeben. Ein weiterer Aspekt der vorliegenden Erfindung betrifft ein Polynukleotid, umfassend mindestens 2 erfindungsgemäße Polynukleotid-Fragmente, wobei die Sequenzen der Polynukleotid-Fragmente auch überlappen oder durch einen Nukleotid-Platzhalter voneinander getrennt sein können. Die Sequenzen der Polynukleotid-Fragmente können identisch oder verschieden sein. Die erfindungsgemäßen Polynukleotide oder Polynukleotid-Fragmente können als Impfstoffe oder Therapeutika oder zur Diagnostik verwendet werden.

Die kodierende Sequenz des Klons 97cn54 und Derivate davon, ausgeführt in Form der SEQ ID NO: 1, als Vertreter des HIV-1 vom Subtyp C kann als Grundlage für die folgenden Anwendungen verwendet werden:

Entwicklung von Subtyp-C-spezifischen HIV-1-Impfstoffen für prophylaktische und therapeutische Zwecke. Diese Subtyp-spezifischen Impfstoffe können weltweit in allen geographischen Regionen, wo das Subtyp-C-Virus für die HIV-Epidemie eine wesentliche Rolle spielt, verwendet werden, also z.B. in Lateinamerika, in Afrika und in Asien. Insbesondere sollten HIV-Impfstoffe, die getestet werden sollen in und entwickelt werden sollen für Südost-Asien und China auf der beschriebenen kodierenden Sequenz von 97cn54 beruhen, um Subtyp-spezifische humorale und zellvermittelte Immunantworten zu induzieren. Desweiteren können solche HIV-1 Subtyp C-spezifischen Impfstoffe als eine Komponente in einer Cocktail-Vakzine eingesetzt werden, die entweder alle oder eine definierte Auswahl der weltweit relevanten HIV Subtypen berücksichtigt.

Um gute humorale und zellvermittelte Immunantworten in den Impflingen zu induzieren, ent-halten die Antigene oder kodierenden Sequenzen, die dem Immunsystem zugeführt werden sollen, vorzugsweise (i) kurze kontinuierliche Abschnitte von mindestens 3 bis etwa 5 Aminosäuren Länge oder längere Abschnitte, abgeleitet von einem der offenen Leserahmen, wie sie in Tabelle 3 abgebildet sind, (ii) Bereiche von vorzugsweise 9 bis 11 Aminosäuren, (iii) Kombinationen dieser Bereiche, die entweder getrennt oder als Polypeptid-Kette (*Epitope-Strings*) verabreicht werden, wobei die Epitope-Strings bzw. deren Aminosäuresequenzen entweder überlappen oder aber durch Aminosäuren oder andere Platzhalter getrennt sein können, und besonders bevorzugterweise vollständige Proteine oder die entsprechenden kodierenden Sequenzen oder deren Varianten, die auch umfangreiche Deletionen umfassen können. Daher betrifft eine andere Aufgabe der vorliegenden Erfindung Polypeptide, die kodiert werden von den Nukleotidsequenzen oder Fragmenten der Nukleotidsequenzen, wie sie in SEQ ID NO: 1, SEQ ID NO: 2 und SEQ ID NO: 3 dargestellt sind. Vorzugsweise umfaßt das Polypeptid eine kontinuierliche Sequenz von mindestens 8 Aminosäuren, vorzugsweise mindestens von 9 bis 11 Aminosäuren, besonders bevorzugterweise von mindestens 15 Aminosäuren oder längere Sequenzen oder diskontinuierliche Epitope, die sich vorzugsweise aus wenigstens drei Aminosäuren einer einzigen Polypeptidkette oder, im Falle oligomerer Proteinkomplexe, auch unterschiedlicher Polypeptidketten zusammensetzen. Impfstoff-Konstrukte auf der Basis der kodierenden Sequenz von 97cn54 schließen alle im Stand der Technik bekannten Antigenformen ein und greifen auf einschlägige Verabreichungssysteme zurück.

Kurze Epitope, kodiert von Fragmenten der Nukleinsäuresequenzen gemäß SEQ ID NO: 1 bis 3, und jeweils drei bis fünf Aminosäuren, vorzugsweise von 9 bis 11 oder mehr Aminosäuren umfassend, können vorzugsweise synthetisch hergestellt werden. Derartige Peptide enthalten entweder ein B-Zellepitop, ein MHC Klasse II-restringiertes T-Helferepitop, ein MHC Klasse I-restringiertes zytotoxisches T-Zellepitop oder Kombinationen der genannten Varianten. Dabei können einzelne Epitope überlappen oder auch durch Platzhalter, präferentiell bestehend aus Glyzin und/oder Serin Resten voneinander getrennnt sein. Verzweigtkettige Peptide können entsprechend dem Stand der Technik entweder während der Synthese oder unter Zuhilfenahme der gängigen und kommerziell erhältlichen homo- und heterobifunktionellen chemischen Quervemetzer im Anschluß an die Synthese und Reinigung der entsprechenden Peptide erzeugt werden. Alternativ können *per se* wenig immunogene Peptide durch Quervernetzung auch an ausgewählte Trägerproteine wie z.B. Ovalbumin konjugiert werden, gentechnisch in Trägerproteine inseriert oder an deren N- bzw. C-Terminus fusioniert werden. Vorzugsweise sind derartige Trägerproteine (i) bei Expression in geeigneten Zellkultursystemen (siehe unten) oder (ii) nach geeigneter Rückfaltung des gereinigten, denaturierten Proteins in der Lage, partikuläre Strukturen auszubilden, bei denen B-Zellepitope vorzugsweise auf der Oberfläche des partikulären Carriers zu liegen kommen. Zahlreiche Beispiele solcher zur Ausbildung partikulärer Strukturen tendierender Polypeptide sind mittlerweile bekannt wie beispielsweise das Hepatitis B-Virus (HBV) Core Antigen (HBcAg), das HBV Oberflächenprotein (HBsAg), das HIV gruppenspezifische Antigen, das Polyomavirus VP1 Protein, das Pappillomvirus L1 Protein oder das TyA Protein der Hefe. Aufgrund der Tatsache, daß sich die Mehrheit der bislang beschriebenen partikelbildenden Proteine aus den Kapsid- oder Strukturproteinen unterschiedlichster Viren rekrutiert, spricht man hier auch von Virus-ähnlichen Partikeln (VLP, virus-like particles; Übersicht: Sonderausgabe Vaccine. (1999) Volume 18. Advances in Peptide, Protein and Nucleic Acid Vaccine Strategies, edited by Pof. P.T.P. Kauyama)

*Epitop-Strings* und Polypeptide, kodiert von Fragmenten der Nukleinsäuresequenzen gemäß SEQ ID NO: 1 bis 3, mit einer Länge größer 30, vorzugsweise größer 50 Aminosäuren sowie Polypeptide mit einer Tendenz zur Ausbildung partikulärer Strukturen (VLP) können nach dem Stand der Technik in Prokaryonten produziert und gereinigt werden. Derartige Plasmide enthalten dementsprechend einen bakteriellen Replikationsursprung wie z.B. ColE1, in aller Regel einen Selektionsmarker wie z.B. eine Resistenz gegenüber Kanamyzin oder Ampizillin, eine konstitutiv aktive oder induzierbare Transkriptions-Kontrolleinheit wie beispielsweise den LacZ- oder Tac Promotor, sowie die Signale zum Translationsstart und Translationsstop. Zur vereinfachten Expression und Affinitätsreinigung können auch optional abspaltbare Fusionsanteile und Reinigungshilfen wie beispielsweise die Glutathion-S-Transferase oder Reinigungshilfen wie z.B. Oligohistidin-*tags* (Fänger) verwendet werden.

Die DNA- oder RNA-Sequenzen, die (i) zur Herstellung der Epitop-*Strings*, kompletter Proteine oder Virus-ähnlicher Strukturen in eukaryontischen Zellkulturen wie z.B. Hefezellen, Pilzen, Insektenzellen oder Säugerzellen verwendet werden oder die (ii) zur direkten Verabreichung von DNA zu Immunisierungszwecken eingesetzt werden, können sich auf eine Verwendung der Kodons verlassen, wie sie vom Virus selbst verwendet wird. Alternativ kann die Verwendung der Kodons, wo immer technisch möglich, angepasst werden an die am häufigsten oder zweithäufigsten verwendeten Kodons in Genen, die im jeweiligen Produktionssystems hoch exprimiert werden. Beispiele für die Optimierung des Kodongebrauchs in einem unter Sicherheitsaspekten optimierten Polygen, beinhaltend die Gene Gag, Pol und Nef, sowie im Hüllprotein-Gen sind gegeben in SEQ ID NO: 2 und SEQ ID NO: 3. Die SEQ IDs NO: 2 und 3 sind in Beispiel 15 näher spezifiziert.

Die Etablierung von Zellinien zur Produktion der *Epitop-Strings,* Polypeptide oder Virus-ähnlichen Strukturen in den genannten Zellkultur-Systemen kann dem Stand der Technik entsprechend auf Vektoren basieren, die wiederum neben einem bakteriellen Replikationsursprung, einem positiven oder negativen Selektionsmarker vor allem die entsprechenden Kontollregionen zur regelkonformen Transkription und Translation des Fremdproteins beinhalten können. Die nachfolgend beschriebenen Komponenten der DNA Vakzinkonstrukte stehen exemplarisch auch für die Module, die sich auch in Vektoren zur Expression der *Epitop-Strings,* Polypeptide oder kompletten Proteine in unterschiedlichen Säugerzellkulturen wiederfinden.

Bei der einfachsten Form der Immunisierung handelt es sich um die direkte Verabreichung eines reinen DNA-Impfstoffes. Dieser enthält im wesentlichen 5'-seitig vom kodierenden Bereich eine Transkriptions-Kontrollegion, auch Promotor/Enhancer-Region genannt, der wahlweise ein funktionelles Intron zur Steigerung der Genexpression folgen kann, (ii) eine Kozak-Sequenz inklusive eines Translations-Startkodons sowie am 3'-Ende des Fremdgens ein Translations-Terminationskodon gefolgt von einer Polyadenylierungs-Signalsequenz. Die Promotor/Enhancerregion kann präferentiell eine konstitutive Expression des gewünschten Genproduktes unterstützen und ist beispielsweise von der Transkriptions-Kontrollregion eines unmittelbar frühen (IE) Cytomegalievirus-Gens (CMV-IE) oder dem Rous-Sarcoma Virus (RSV) LTR (*long terminal repeat*) abgeleitet. Alternativ kann auch eine induzierbare Form einer Transkriptions-Kontrollregion wie z.B. ein *Tet on*/*Tet off* Promotor verwendet werden, bei dem die Transkription beispielsweise durch die Gabe von Tetrazyklin oder entsprechender Analoga reguliert wird. Desweiteren bietet sich hier die Verwendung von Zelltyp-spezifisch regulierten Transkriptions-Kontrollregionen an wie z.B. die stromaufwärts des Muskel-Kreatin-Kinase Gens (MCK Gen; muskelspezifische Expression), des CD4-Rezeptorgens oder der MHC Klasse II Gene (präferentielle Expression in Antigen-präsentierenden Zellen) gelegenen Promotor/Enhancerregionen. In einigen Fällen verwendet man auch chimäre Kombinationen aus (i) Zelltyp-spezifischen Promotoren und (ii) viralen Enhancerregionen, um die Vorteile einer gewebespezifischen Expression mit denen der starken Transkriptionsaktivität viraler Enhancer zu vereinen. Die Verstärkung der Genexpression durch das Einbinden eines in aller Regel 5'-seitig des offenen Leserahmens gelegenen funktionellen Introns geht auf eine gesteigerte Kemexportrate gespleißter im Vergleich zu ungespleißten Transkripten zurück und wird beispielsweise durch die Insertion eines im β-Globin Gen gelegenen Introns erreicht.

Eine bevorzugte Form eines auf SEQ ID NO:1, 2 oder 3 basierenden DNA-Impfstoffs enthält zusätzlich ein von Alpha-Viren wie beispielsweise von Semliki-Forest- oder Venezuela-Encephalitis-Viren (SFV, VEE) abgeleitetes Replikon. In diesem Fall folgt der oben beschriebenen nukleären Transkriptions-Kontrolleinheit und dem wahlweise berüchsichtigten Intron zunächst der für die VEE oder SFV Nichtstrukturproteine (NS) kodierende Bereich. Erst 3' seitig davon folgt das eigentliche Fremdgen, dessen zytoplasmatische Transkription seinerseits durch einen NS-sensitiven Promotor reguliert wird. Dementsprechend wird ausgehend von der nukleären Transkriptions-Kontrolleinheit ein langes Transkript über mehrere offene Leserahmen erzeugt, das anschließend ins Zytoplasma transloziert wird. Die dort synthetisierten NS-Proteine aktivieren dann durch Bindung an die entsprechende Kontrollregion die zytoplasmatische Transkription der Fremdgene. Dieser Amplifikationseffekt führt in aller Regel zu einer abundanten RNA Synthese und folglich hohen Fremdprotein-Syntheseraten. Letzteres erlaubt, im direkten Vergleich mit konventionellen Plasmiden, die auf den beschriebenen Effekt durch zytoplasmatische RNA Amplifikation verzichten, in aller Regel eine deutliche Reduktion der zu verabreichenden Plasmidmenge bei wenigstens vergleichbarer Immunogenität.

Die oben beschriebenen Peptide, Proteine, Virus-ähnlichen Partikel und DNA-Konstrukte können durch intramuskuläre, subkutane, intradermale, intravenöse Injektion verabreicht werden, wobei für die Verabreichung der proteinösen Antigene jeweils der Stand der Technik angewendet wird. Zur DNA-Immunisierung können entweder konventionelle Spritzen mit Injektionsnadeln verwendet werden, oder aber Gerätschaften, die ohne Nadeln auskommen und in aller Regel die DNA über Druckluft direkt in das gewünschte Gewebe einbringen können. Dazu zählt insbesondere auch die intranasale und orale Applikation DNA-haltiger Vakzin- Formulierungen durch sprayartige Vorrichtungen. Alternativ dazu kann die DNA auch an feste Träger wie z.B. Goldkügelchen konjugiert und beispielsweise unter Luftdruck in die entsprechenden Gewebe verabreicht werden.

Zur Verstärkung oder Modulation der Immunantwort können die erwähnten proteinösen Antigene und DNA-Konstrukte auch mit sogenannten Adjuvantien, i.d. Regel Stimulatoren der Immunantwort, kombiniert oder in einer sequentiellen Abfolge mit den Adjuvantien verabreicht werden. Konventionelle Adjuvantien wie z.B. Aluminiumhydroxyd oder Aluminium-Hydroxyphosphat resultieren in einer Stimulation der humoralen Immunantwort, die sich auch durch hohe Antikörpertiter vom IgG1 Subtyp auszeichnet. Modernere Adjuvantien, wie beispielsweise CpG Oligonukleotide (Konsensuskemmotiv: Purin-Purin-CpG-Pyrimidin-Pyrimidin) oder chemisch modifizierte Derivate davon (Phosphothioat-Oligunukleotide; Oligonukleotide mit Peptidrückgrat) verstärken üblicherweise den zellulären Arm der Immunantwort und unterstützen vornehmlich den Th1-Typ der zellvermittelten Immunität, gekennzeichnet durch hohe Antikörpertiter vom Subtyp IgG2a und die Induktion von Th1 Zytokinen wie z.B. γ-IFN, IL-2 und IL-12.

Die Verabreichung und Aufnahme von Peptiden, Proteinen und DNA-Vakzinkonstrukten kann insbesondere auch verbessert werden durch Bindung an oder Inkorporation in höhermolekulare Strukturen, wie z.B. biodegradierbare Partikel, multilamellare, idealerweise kationische Liposomen, immunstimulierende Komplexe (ISCOMS), Virosomen oder *in vitro* assemblierter Viruspartikel. Zu biodegradierbaren Partikeln zählen beispielsweise PLA- (L-lactic acid), PGA- (polyglycolic) oder PLGA- [poly (D,L-lactide-co-glycolide)] Mikrosphären oder Derivate davon, kationische Mikropartikel oder von bakteriellen Kapselpolysacchariden abgeleitete Trägersubstanzen. Der Sammelbegriff ISCOMS steht für immunstimulierende Komplexe, die auf wasserlöslichen Extrakten der Rinde von *Quillaja saponaria* entstammen und mittels chromatographischer Verfahren weiter aufgereinigt wurden. Eine dem Stand der Technik entsprechende, detaillierte Übersicht zu den unterschiedlichsten Adjuvantien und Verabreichungshilfen findet sich unter http://www.niaid.nih.gov/aidsvaccine/pdf/compendium.pdf [Vogel, F. R., Powell, M. F. and Alving, C. R. "A Compendium of Vaccine Adjuvants and Excipients (2nd Edition)].

Desweiteren können zur günstigen Präsentation von *Epitop-Strings,* Polypeptiden und Virus-ähnlichen Partikeln virale und, alternativ, bakterielle Vektoren eingesetzt werden.

Nach dem aktuellen Stand der Technik eignen sich beispielsweise gentechnisch veränderte Salmonellen und Listerien aufgrund ihres natürlichen Zelltropismus in besonderer Weise dazu, DNA-Vakzinkonstrukte in Antigen- präsentierende Zellen wie Monozyten, Makrophagen und vor allem in dendritische Zellen einzubringen. Die gentechnischen Veränderungen können neben einem Gewinn an Zelltypspezifität unter anderem dazu beitragen, daß die DNA unbeschadet das Zytoplasma der Antigen-präsentierenden Zelle erreicht. In diesem Fall gelangt ein DNA-Vakzinkonstrukt in den Zellkern, wo über einen eukaryontischen, vorzugsweise viralen oder zelltypspezifischen Promotor der entsprechende Leserahmen unter Nutzung der zellulären Resourcen und Proteine transkribiert wird. Nach dem Transport der RNA ins Zytoplasma wird das entsprechende Genprodukt translatiert und, je nach Beschaffenheit, posttranslational modifiziert und dem entsprechenden zellulären Kompartiment zugewiesen.

Bakterielle Vektoren (Salmonellen, Listerien, Yersinien etc.) können auch zur Induktion einer Schleimhautimmunität, vorzugsweise nach oraler Verabreichungverwendet werden Dabei werden die entsprechenden Antigene durch die bakterielle Transkriptions- und Translationsmaschinerie hergestellt und unterliegen demnach nicht den in Säugerzellen sonst üblichen posttranslationalen Modifikationen (keine entsprechende Glykosylierung; kein sekretorischer *Pathway*).

Daneben existieren mittlerweile eine Vielzahl von attenuierten viralen Vektoren, mit deren Hilfe sich die gewünschten Antigene erfolgreich und in hohen Ausbeuten exprimieren lassen. Neben deren Tauglichkeit zur reinen Antigen-Produktion können solche virale Vektoren auch direkt zur Immunisierung eingesetzt werden. Diese kann zunächst entweder *ex vivo* erfolgen, beispielsweise zur Infektion von Antigen-präsentierenden Zellen, die anschließen dem Impfling verabreicht werden, oder direkt *in vivo* durch die subkutane, intradermale, intracutane, intramuskuläre oder intranasale Immunisierung mit dem rekombinanten Virus. die eine günstige Antigen-Präsentation mit entsprechendem Immunisierungserfolg erzielen läßt. So können beispielsweise durch Immunisierung mit rekombinanten Vakzinia Viren wie z.B. dem durch Passagieren über Hühnerzellen attenuierten *Modifizierten Vaccinia Virus Ancara* (MVA), dem gentechnisch attenuierten Vaccinia Stamm *New York* (NYVAC) oder die in Vögeln endemischen aviären Vaccinia Viren (*Fowlpox, Canarypox*) adäquate humorale und zellvermittelte Immunantworten in den geimpften Personen induziert werden. Alternativ eignen sich dazu in gleicher Weise auch eine Reihe anderer Viren wie z.B. rekombinante Alpha-Viren, darunter das Semliki-Forest Virus oder das Venezuela-Enzephalitis Virus, rekombinante Adenoviren, rekombinante Herpes Simplex Viren, Influenzaviren und andere.

Letztlich können auf Basis der SEQ ID NO:1, 2, oder 3 auch attenuierte HIV-Viren generiert und zu Immunisierungszwecken eingesetzt werden, sofern mittels Klonierverfahren nach dem Stand der Technik die Regulationssequenzen (LTR, long terminal repeat), die den kodierenden Bereich flankieren, ergänzt werden. Eine hinreichende Attenuierung des Virus kann dann entsprechend dem Stand der Technik durch eine oder mehrere Deletionen beispielsweise im Nef-Gen erzieltwerden

Die in den Beispielen SEQ ID NO: 1 und SEQ ID NO: 3 ausgeführten Nukleinsäuresequenzen sowie daraus abgeleitete Peptide, Proteine oder Virus-ähnlichen Partikel können auch als Komponenten viraler Vektoren zur Genüberführung eingesetzt werden.

Die durch das GagPol-Gen (SEQ ID NO:1; Nukleotid 167-4458; Tabelle 3) kodierten Polypeptide können beispielsweise die Verpackungs- und Rezeptorfunktionen von z.B. lenti- oder retroviralen Vektoren bereitstellen. So können z.B. nach transienter Transfektion von Säugerzellen durch geeignete Plasmidvektoren, welche die gleichzeitige Expression des GagPol und VSV-G (vesicular stomatitis virus Hüllprotein G) Gens unterstützen und die Verpackung eines therapeutischen Transgens sicherstellen, Viruspartikel erzeugt werden, die auch in der Lage sind, ruhende, postmitotische oder enddifferenzierte Zellen zu transduzieren. Dieses Verfahren zur Generierung transduktionskompetenter Viruspartikel kann wesentlich erleichtert und effizienter gestaltet werden, beispielsweise durch die Etablierung stabiler Zellinien, z.B. basierend auf *human embryonic kidney* Zellen (HEK293), die das GagPol Polyprotein konstitutiv oder unter Kontrolle eines induzierbaren Promotors exprimieren. Alternativ können auch rekombinante Adenoviren generiert werden, die die Verpackungsfunktionen, die Rezeptorfunktionen und die Transgenfunktionen oder Kombinationen daraus kodieren, und so als Werkzeug zum *ex vivo, in situ* und *in vivo* Delivery von retro- oder lentiviralen Vektoren dienen.

Die durch SEQ ID NO: 3 kodierten Hüllproteine oder Derivate davon können die Rezeptorfunktion für lenti-, spuma- oder retrovirale Vektoren oder anderer, auf umhüllten Viren basierender Vektoren durch Inkorporation in den Lipid-Bilayer bereitstellen. Dazu können beispielsweise auch Verpackungslinien erzeugt werden, bei denen sowohl die GagPol Proteine von Retro-, Spuma- und vorzugsweise von Lentiviren, als auch die aus SEQ ID NO: 1 und 3 abgeleiteten Hüllproteine entweder konstitutiv oder unter Kontrolle eines induzierbaren, wahlweise eines in der Aktivität regulierbaren Promotors exprimiert werden. Alternativ dazu können, beispielsweise basierend auf dem Genom von Typ C oder Typ D Retroviren oder anderer membranumhüllter Viren wie z.B. Influenza- oder Herpesviren, chimäre Viren generiert werden, die zusätzlich zu dem natürlichen Hüllprotein oder anstelle des natürlichen Hüllproteins ein von SEQ ID NO: 1 oder SEQ ID NO: 3 abgeleitetes Hüllprotein auf der Oberfläche tragen.

Gegen die aus den SEQ IDs NO: 1 bis 3 abgeleiteten Peptide, Proteine oder Virus-ähnlichen Partikel können auch (i) polyklonale Antiseren, (ii) monoklonale Antikörper (Maus, Mensch, Kamel), (iii) Antikörperderivate wie beispielsweise *single-chain* Antikörper, humanisierte Antikörper, bispezifische Antikörper, Phagen-Antikörperbanken oder (iv) andere hochaffin bindende Polypeptide wie z.B. Derivate des hPSTI (human pancreatic secretory trypsin inhibitor) generiert werden. Diese Reagentien können zu therapeutischen Zwecken, beispielsweise zur Behandlung von HIV-Infektionen oder zu diagnostischen Zwecken, beispielsweise zur Herstellung von Testkits verwendet werden.

Auf ähnliche Weise können die aus SEQ ID NO:1, 2 oder 3 abgeleiteten Peptide, Proteine oder Nukleinsäure-Sequenzen für diagnostische Zwecke, z.B. für die Serodiagnostik und für die Anwendung von Nukleinsäure-Hybridisierungstechniken oder Nukleinsäure-Amplifikationssystemen oder Kombinationen davon verwendet werden. Vorzugsweise können die erfindungsgemäßen Polynukleotid-Fragmente der Nukleotidsequenz gemäß SEQ ID NO: 1 in einer Polymerase-Kettenreaktion eingesetzt werden. Besonders bevorzugt werden die erfindungsgemäßen Polynukleotid-Fragmente der Nukleotidsequenz gemäß SEQ ID NO:1 zur Diagnostik mittels DNA-Chiptechnologie eingesetzt.

Die Erfindung wird durch die nun folgenden Beispiele erläutert, ist aber nicht auf diese beschränkt:

### Beispiel 1:

### Blutproben

Alle Blutproben, die für diese Studie verwendet wurden, wurden im Zuge der nationalen, molekularepidemiologischen Studie von 1996/1997 bezüglich HIV-1, Subtyp C, seropositiven IDUs aus mehreren HIV-epidemischen Gebieten in China entnommen. Periphere mononukleäre Blutzellen (PBMC) wurden mittels Ficoll-Gradienten abgetrennt. Die Viren wurden durch Kokultivierung der PBMCs von seropositiven IDUs mit Phytohämaglutinin (PHA)-stimulierten Donor-PBMCs isoliert. Positive Viruskulturen wurden aus den Zellkultur-Überständen nachgewiesen mittels des HIV-1 p24 Core Profile ELISA-Kits (DuPont Inc., Boston, MA).

### Beispiel 2:

### Polymerase Kettenreaktionen (PCR) und DNA-Sequenzierung

Provirale DNA wurde aus produktiv infizierten PBMCs von mehr als einhundert ausge-wählten HIV-1-positiven IDUs aus den nordwestlichen Provinzen Chinas extrahiert (Qiagen Inc., Valencia, CA). Die *Nested*-PCR wurde verwendet, um die kodierende Region für env C2V3 zu amplifizieren. Die PCR-Produkte wurden mittels der *Taq-cycle-*Methode unter Ver-wendung von Fluoreszenzfarbstoff-markierten Terminatoren (Applied Biosystems, 373A, Foster City, CA) wie kürzlich beschrieben (Bai et al. 1997; Yu et al. 1997) direkt sequenziert. Multiple Sequenzvergleiche wurden unter Verwendung der *Wisconsin software package Genetics Computer Group* mit den Korrekturmethoden nach Kimura durchgeführt (GCG, 1997, Version 9).

### Beispiel 3:

Phylogenetische Stammbaum-Analysen wurden von allen erhaltenen Sequenzen unter Verwendung des PHYLIP-Software-Pakets durchgeführt. Evolutionäre Entfernungen wurden an Hand der *maximum parsimony*-Methode berechnet und durch kumulative horizontale Länge der Zweige angegeben. Die statistische Robustheit des *neighbour joining* Stammbaums wurde wie kürzlich beschrieben durch *bootstrap resampling* überprüft (Graf et al. 1998).

### Beispiel 4:

### Auswahl eines repräsentativen HIV-1-Isolats des Subtyps C von chinesischen IDUs

Innerhalb der Gruppen betrugen die berechneten durchschnittlichen Abstände innerhalb der für C2V3 kodierenden Region auf DNA-Ebene 2,26 ± 1,43, was darauf hindeutet, daß die Epidemie in diesem Gebiet noch sehr jung ist. Die Unterschiede zwischen den Gruppen zwischen chinesischen Subtyp-C-Sequenzen und denen aus Indien, Afrika, und Südamerika betrugen 9,67 ± 2,31 (Indien), 15,02 ± 4,13 (Afrika) und 8,78 ± 3,41 (Südamerika). Das zeigt eine enge phylogenetische Verwandtschaft zwischen indischen und chinesischen Subtyp-C-Sequenzen (Lole et. al. 1999) und eine nennenswerte genetische Entfernung zu der *per se* relativ heterogenen Gruppe afrikanischer HIV-1-Stämme des Subtyps C.

### Beispiel 5:

### Identifizierung eines Virus-Isolats, das den in China zirkulierenden prävalenten Virus-Stamm des Subtyps C am besten repräsentiert

Aus den analysierten Proben wurde ein als 97cn54 bezeichnetes repräsentatives Isolat identifiziert, das höchste Homologie (99,6%) zu einer berechneten Konsensus-Sequenz (cnconV3), die auf Grundlage der charakterisierten lokalen HIV-Sequenzen (Tabelle 1) erstellt worden ist, aufweist. Multiple Aminosäure-Sequenzvergleiche einschließlich der Primärsequenzen der V3-Schleife von primären Subtyp-C-Vertretern aus den verschiedensten epidemischen Regionen und auch Konsensus-Sequenzen von anderen Subtypen (A-H, O, CPZ) unterstrichen den Subtyp-C-Charakter des ausgewählten Primärisolats 97cn54 (Tabelle 1). Verglichen mit einer V3-Gesamtkonsensus-Sequenz (consensus) zeigen sowohl 97cn54 als auch cn-con-c Aminosäure-Abweichungen an den Positionen 13 (H→R) und 19 (A→T), die beide charakteristisch für Isolate des Subtyps C sind (C_consensus).

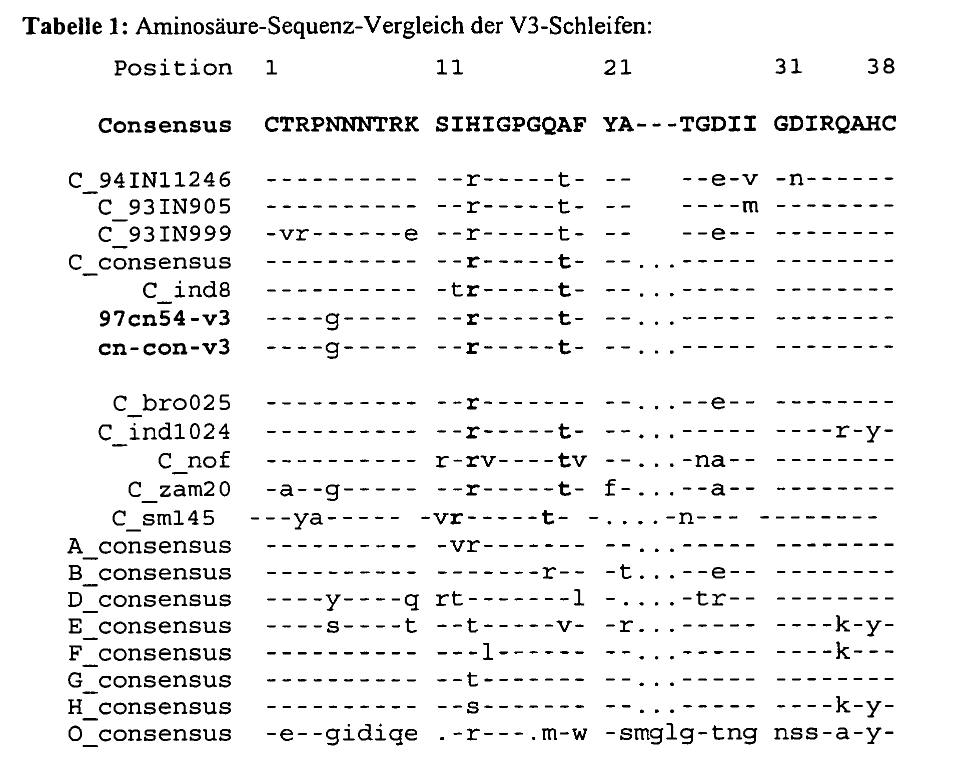

**Tabelle 1:** Der Aminosäure-Sequenz-Vergleich der V3-Schleifen von Konsensus-Sequenzen verschiedener Subtypen von HIV-1 (A-O) und ausgewählte Isolate des Subtyps C aus verschiedenen Ländern. Die V3-Gesamtkonsensus-Sequenz wurde durch den Vergleich der Konsensus-Sequenzen von verschiedenen Subtypen (A-O) ermittelt. cn-con-V3 stellt die Konsensus-Sequenz von HIV-1-Stämmen Subtyps C, die in China prävalent sind, dar. 97cn54 wurde als repräsentatives Standard-Isolat der in China vorkommenden prävalenten HIV-1-Stämme des Subtyps C ausgewählt. "-" bedeutet keinen Austausch gegenüber der V3-Konsensus-Sequenz, Kleinbuchstaben bedeuten eine Aminosäure-Substitution und "." bedeutet Lücken. Alle Konsensus- und Isolat-Sequenzen für multiple Vergleiche wurden von der Datenbank Los Alamos erhalten.

### Beispiel 6:

Die für das 97cn54 Hüllprotein kodierende Sequenz ist am nächsten verwandt mit Virus-Stämmen des Typs C aus Indien.

Phylogenetische Stammbaum-Analysen, ursprünglich basierend auf den C2V3-Sequenzen des env-Gens, ergaben, daß sowohl 97cn54 als auch die Konsensus-Sequenz der chinesischen Isolate des Subtyps C sich mit den Stämmen des Subtyps C aus Indien (ind8, d1024, c-93in905, c-93in999, c-93in11246), aus Afrika (c-eth2220, c-ug286a2), und aus Südamerika (92br025, nof, cam20 und sm145) gruppieren (clustern). Dies weist darauf hin, daß die indischen Virus-Stämme des Subtyps C der Ursprung der Epidemie von HIV-1, Subtyp C, in China sein könnten (Figur 1). Diese Hypothese stimmt auch überein mit unserer früheren epidemiologischen Erkenntnis, die bestätigt, daß mit HIV-1, Subtyp C, infizierte Menschen in Yunnan Injektionskanülen mit indischen Schmuckhändlern im Grenzgebiet geteilt haben sollen (Shao et al. 1999).

### Beispiel 7:

### Klonierung des HIV-1-Genoms von im wesentlichen voller Länge

Genome von HIV-1 von im wesentlichen voller Länge wurden amplifizeirt mittels des *Expand Long Template* PCR-Systems (Boehringer-Mannheim, Mannheim, Deutschland), wie beschrieben bei Graf et al. (1998) und Salminen et al. (1995). Die Startermoleküle (Primer) wurden in konservierten Regionen innerhalb der langen terminalen Wiederholungen (LTR) von HIV-1 positioniert: TBS-A1 (5'-ATC TCT AGC AGT GGC GGC CGA A) und NP-6 (5'-GCA CTC AAG GCA AGC TTT ATT G). Gereinigte PCR-Fragmente wurden mit glatten Enden in einen mit *SrfI* verdauten pCR-Script-Vektor (Stratagene, Heidelberg, Deutschland) ligiert und in den E. *coli*-Stamm DH5α transformiert. Verschiedene rekombinante Klone, die im wesentlichen das HIV-1-Genom voller Länge enthielten, wurden mittels Restriktionsfragmentlängen-Polymorphismus (RFLP) und Sequenzierung der kodierenden Sequenz der V3-Schleife identifiziert. Laut RFLP-Analyse unter Verwendung verschiedener Kombinationen von Restriktionsendonukleasen und nachfolgender Sequenzierung der kodierenden Sequenz der V3-Schleife waren 77% der positiven Konstrukte voller Länge nahezu identisch. Ein Provirus-Konstrukt, das die breite Mehrheit der positiven Klone repräsentiert, wurde ausgewählt und wie oben beschrieben unter Verwendung des *primer-walking*-Ansatzes sequenziert (die Startermoleküle wurden ungefähr alle 300 bp entlang des Genoms für beide Stränge entworfen).

### Beispiel 8:

DNA-Sequenzen wurden unter Verwendung der Lasergene Software (DNASTAR, Inc., Madison, WI) auf Macintosh-Computern zusammengesetzt. Alle Referenzsequenzen der Subtypen dieser Studie sind von der Los Alamos HIV Datenbank. Ähnlichkeiten in der Nukleotid-Sequenz wurden mittels des lokalen Homologie-Algorithmus von Smith und Waterman berechnet. Multiple Sequenzvergleiche mit verfügbaren Sequenzdaten anderer Subtypen wurden unter Verwendung des Wisconsin Softwarepakets *Genetics Computer Group* (GCG, 1997, Version 9) durchgeführt.

### Beispiel 9:

### Gesamtstruktur der kodierenden Sequenz von 97cn54

Die 9078 bp lange genomische Sequenz des Isolats 97cn54 enthielt alle bekannten strukturellen und regulatorischen Gene des HIV-1-Genoms. Es wurden keine wesentlichen Deletionen, Insertionen oder Umlagerungen gefunden. Die Ähnlichkeiten in der Nukleotid-Sequenz wurden untersucht mittels Vergleich aller kodierenden Sequenzen (CDS) von 97cn54 mit Konsensus-Sequenzen verschiedener Genotypen und ausgewählter Subtyp-Isolate (Tabelle 2). Die höchsten Homologien der Leserahmen von gag, pol, env und vif zu den entsprechenden Konsensus-Sequenzen des Subtyps C lagen in einem Bereich von 93,93 bis 95,06%. Diese Beobachtung erweiterte den oben beschriebenen Sequenzvergleich und die phylogenetische Stammbaum-Analyse aufgrund von C2V3 erheblich (siehe Tabelle 1 und Figur 1). Sie bestätigte daher eindeutig, daß das ausgewählte Virus-Isolat zur Gruppe der kürzlich veröffentlichten Virus-Stämme des Subtyps C gehört. Die durch diese Art der Analyse für die Gene tat, vpu, vpr und nef bestimmten Werte der Homologie waren jedoch nicht ausreichend, um eine klare Zuordnung dieser Leserahmen zu Virus-Stämmen des Subtyps B oder C zu erlauben (Tabelle 2). Für das Gen vpu wurden die höchsten Homologien zu den Subtypen B registriert (94,24%), während die Homologie zu der Konsensus-Sequenz des Subtyps C nur 78,23% betrug. Ähnliche Beobachtungen wurden für das Gen tat gemacht: höchste Homologie zum Isolat B'-r142 (>91%), im Vergleich zu 87,9% (C-92br025) und 85,5% (C-eth2220) für ausgewählte primäre Vertreter des Subtyps C oder 89,01% für die Konsensus-Sequenz des Subtyps C. Diese Daten legten zusammen mit dem Auftreten der Genotypen B, C und E im ganzen epidemischen Gebiet von Yunnan nahe, daß das analysierte Virus-Isolat einen Mosaik-Virusstamm darstellen könnte, der die Folge eines Rekombinationsvorgangs zwischen Subtyp B' und Subtyp C ist.

**Tabelle 2: Vergleich der kodierenden Sequenzen von 97cn54 mit den entsprechenden Genen von Referenz-Stämmen und Subtyp-spezifischen Konsensus-Sequenzen.**

| Prozent Identität mit 97cn54 | | | | | | | | | |
|---|---|---|---|---|---|---|---|---|---|
| **CDS** | **gag** | **pol** | **vif** | **vpr** | **tat** | **rev** | **vpu** | **env** | **nef** |
| A | 87.68 | 91.80 | 86.81 | 83.66 | 84.90 | 83.97 | 79.82 | 85.75 | 84.19 |
| B | 90.43 | 91.93 | **88.04** | **90.31** | **86.56** | 82.08 | 94.24 | 84.52 | **88.13** |
| B-mn | 89.38 | 90.82 | **86.01** | **89.31** | **87.44** | 79.48 | 88.21 | 82.33 | **85.41** |
| B'-r142 | 91.53 | 90.76 | **86.01** | **88.97** | **91.163** | 80.23 | 96.74 | 82.70 | **85.99** |
| C | 94.65 | 94.29 | **95.06** | **91.39** | **89.01** | 91.99 | 78.23 | 93.93 | **88.82** |
| C-92br025 | 92.19 | 92.91 | **88.51** | **90.03** | **87.91** | 89.70 | 76.13 | 88.51 | **86.20** |
| C-eth2220 | 91.4 | 92.06 | **87.15** | **90.77** | 85.57 | 88.08 | 80.09 | 87.15 | 87.08 |
| D | 89.80 | 91.08 | 87.74 | 87.94 | 83.93 | 84.39 | 87.30 | 85.26 | 86.88 |
| E/A | 86.324 | 89.07 | 86.59 | 83.39 | 81.44 | 81.74 | 77.31 | 82.09 | 84.18 |
| F | 88.02 | 88.99 | 86.36 | 86.25 | 80.65 | 86.25 | 82.33 | 84.02 | / |
| G | 88.08 | / | / | / | / | / | / | 84.55 | / |
| H | 87.69 | 89.45 | 86.01 | 85.22 | / | / | / | 83.74 | / |
| O | 73.42 | 78.02 | 72.12 | 76.604 | 72.31 | 76.60 | 59.54 | 67.01 | 80.35 |
| CPZ | 74.14 | 78.80 | 93.75 | 75.44 | 76.00 | 75.44 | 64.41 | 72.42 | / |

**Tabelle 2:** Nukleotidsequenz-Vergleich aller kodierenden Sequenzen (CDS) zwischen 97cn54 und DNA-Sequenzen, die entweder (1) Konsensus-Sequenzen bestimmter HIV-1-Subtypen (erhalten von der Los Alamos HIV-Datenbank) oder (2) Isolate des Standard-Subtyps C (92br025 und eth2220) und B (mn und r142) darstellen. Die Daten geben die Identität einer bestimmten Sequenz mit 97cn54 in Prozent an. Nicht-eindeutige Nukleotid-Positionen innerhalb der Konsensus-Sequenzen wurden als identisch bewertet. Die höchsten Homologien sind in Fettdruck hervorgehoben. "/" bedeutet, daß von der Los Alamos Datenbank keine Konsensus-Sequenz verfügbar war.

### Beispiel 10:

### Bestimmung der Rekombinationen zwischen den Subtypen

Das rekombinante Identifikationsprogramm (RIP, Version 1.3; http://hiv-wew.lanl.gov/tools) wurde verwendet, um potentielle Mosaik-Strukturen innerhalb der Gesamtsequenz dieses Klons zu identifizieren (Fenstergröße: 200; Schwellenwert für die statistische Signifikanz: 90%; Umgang mit Lücken: STRIP; informativer Modus: OFF). Es wurden Lücken eingerührt, um den Vergleich zu ermöglichen. Die Hintergrund-Sequenzen der Subtypen in dieser Analyse waren: u455 (Subtyp A), RL42 (chinesischer Subtyp B-Thai (B')), eth2220 (Subtyp C), z2d2 (Subtyp D), 93th2 (Subtyp A/E).

### Beispiel 11:

### Rekombination zwischen den Subtypen in der kodierenden Region für Gag-Pol von 97cn54

Auch wenn wesentliche Homologien zu den Virusstämmen des Subtyps C innerhalb der hochkonservierten Leserahmen von gag und pol beobachtet wurden, identifizierte die RIP-Analyse 3 Bereiche der intra-subtypischen Rekombination innerhalb gagpol um die Positionen 478-620, 1290-1830 und 2221-2520 oberhalb des Startkodons von gag. Diese verstreuten Abschnitte liegen innerhalb der Leserahmen von gag und pol und weisen höchste Homologien zu dem Prototyp B (Daten nicht gezeigt) und insbesondere zu einem Isolat des Subtyps B(B'), das aus Yunnan kommt (Figur 2), auf. Diese Beobachtung unterstreicht eindeutig die Wichtigkeit von RIP-Analysen, da einfache Homologie-Vergleiche auf der Basis von kompletten Genen nicht in der Lage waren, diese kleinen verstreuten Fragmente eines anderen Subtyps zu identifizieren. Um die mittels RIP-Analyse erhaltenen Daten zu bestätigen, erstellten wir mehrere phylogenetische Stammbäume unter Verwendung der Regionen, die die Bereiche der vorgeschlagenen Rekombination entweder flankieren oder überspannen (Figur 3). Unter Verwendung mehrerer Standard-Vertreter verschiedener Subtypen und einiger ausgewählter Primär-Isolaten des Subtyps C konnten alle vorgeschlagenen Bereiche der Rekombination bestätigt werden durch differenzielles Clustern von 97cn54 mit den jeweiligen Referenz-Isolaten der Subtypen C (Figuren 3 A, C, E, G) oder B (Figuren 3 B, D, F).

### Beispiel 12:

### Intersubtyp-Rekombination in der für env kodierenden Region von 97cn54

Wie die in Tabelle 2 zusammengefassten Sequenzvergleiche erwarten ließen, bestätigte die RIP-Analyse die Intersubtyp-Rekombination zwischen Subtyp (B')-Thai und C (Figur 4) eindeutig. Ein Fragment von etwa 1000 bp Länge, das sich von den 150 3'-terminalen bp von vpr über das erste Exon von tat und rev bis zu vpu erstreckt, zeigte das höchste Ausmaß an Homologie mit dem Vertreter des lokalen Subtyps (B') (r142) (Figur 4 A). Darüber hinaus zeigte ein etwa 300 bp langer Sequenzbereich, der mit der 5'-Hälfte des Gens nef überlappt, höchste Homologie mit dem Subtyp (B')-Thai, wohingegen der verbleibende Teil einschließlich eines Fragments von 300 bp Länge, der sich in die 3'-LTR-Region erstreckt, mit Subtyp C gruppiert (clustert) (Figur 4 B).

Unter Erweiterung der RIP-Analyse zeigten phylogenetischen Stammbäume die engste Verwandtschaft von vpr/vpu und dem 5'-Bereich des nef-Gens zu Isolaten des Subtyps B (Figur 5 A, B), wohingegen das 3'-nef-Fragement sich eindeutig mit Vertretern des Subtyps C gruppierte (Figur 5 C). Weitere Analysen bestätigten, daß die Sequenz des Subtyps B innerhalb dieses Mosaiks näher verwandt ist mit einem kürzlich beschriebenen Thai-(B')-Stamms (r142), der isoliert wurde von einem chinesischen IDU (Graf et al. 1998), als zu Prototyp-Isolaten des Subtyps B (mn und sf2) (Tabelle 2).

### Beispiel 13:

### Repräsentativer Charakter von 97cn54

In den kodierenden Regionen von vpr/vpu und dem nef-Gen von 97cn54 liegende Bruchstellen wurden in fast identischen Postionen bei allen Stämmen des Subtyps C, die aus in den nordwestlichen Provinzen von China lebenden IDUs isoliert wurden, gefunden. 2 RIP-Analysen, die repräsentativ für 8 unabhängig voneinander isolierte und analysierte HIV-1-Stämme von verschiedenen mit HIV-1 infizierten Personen in der autonomen Region Xinjiang isoliert wurden, sind in den Figuren 4 C und D dargestellt. Was die Herkunft von 97cn54 (Südwesten von China) und xj24 und xj15 (nordwestliches Gebiet) betrifft, legen diese Daten für die durch China zirkulierenden C/B'-rekombinanten Stämme einen gemeinsamen Vorläufer nahe. Unsere Ergebnisse zeigen also, daß 97cn54 ein C/(B')-Intersubtyp-Mosaikvirus mit 10 Bruchstellen der Intersubtyp-Rekombination darstellt, das unter den IDUs innerhalb der nordwestlichen Provinzen Chinas am stärksten prävalent ist. Eine schematische Darstellung des (B'/C)-Mosaikgenoms von Isolat 97cn545 ist in Figur 6 dargestellt.

### Beispiel 14:

### Vorhersage der über Subtypen hinaus kreuzreaktiven spezifischen Epitope für HIV-spezifische zytolytische T-Zellen

Genomische Sequenzen eröffnen die Möglichkeit, die Konserviertheit von bekannten CTL-Epitopen zu ermitteln, die einen Einfluß haben können auf die Effektivität von HIV-1-Impfstoffkandidaten. Die meisten Reagenzien und Daten bezüglich CTL-Epitopen stammen von Sequenzen von HIV-1_{LAI} des Subtyps B. Um die Konserviertheit von über Subtypen hinaus kreuzreaktiven CTL-Epitopen abzuschätzen, wurden die vorhergesagten Protein-Sequenzen von 97cn54 mit den bekannten und am besten kartierten LAI-spezifischen CTL-Epitopen verglichen. Von den 194 beschriebenen CTL-Epitopen von HIV-1 liegen 75, 55, 40 und 24 in Gag, (p17, p24, p15), in der Reversen Transkriptase (RT), in gp120 bzw. in gp41. Während fast 50% oder mehr der Epitope in Gag und RT völlig identisch sind, stimmten nur 5% und 17% der von HN-1_{LAI} abgeleiteten CTL-Epitope von gp120 und gp41 exakt mit der für 97cn54 vorhergesagten Aminosäure-Sequenz überein. Wenn man jedoch zwei konservative Fehlpaarungen in einem bestimmten CTL-Epitop zuläßt, war ein zusätzlicher Bereich von 48% (p17), 33% (p24), 40% (RT), 57% (gp120) und 33% (gp41) der bekannten CTL-Epitope von HIV-1_{LAI} verwandt mit den Sequenzen in den entsprechenden von 97cn54 abgeleiteten Polypeptiden. Natürlich muß diese letzte Betrachtung mit einiger Vorsicht aufgenommen werden, da sogar nicht-konservative Austausche die HLA-Bindung oder die T-Zell-Rezeptorerkennung eines antigenen Peptids beseitigen kann. Zusammengenommen sagen diese Beobachtungen jedoch eindeutig eine beträchtliche über die Subtypen hinaus kreuzreaktive CTL-Reaktivität voraus, insbesondere der funktionell und immunologisch konservierten Proteine von HIV-1. Außerdem legen diese Daten nahe, daß ein beträchtlicher Anteil der Reagenzien (Peptide, Vakziniavirus-Konstrukte), die für die Kartierung und Charakterisierung von CTL-Epitopen des Subtyps B synthetisiert und etabliert worden sind, auch nützlich sein können für die Bestimmung von CTL-Reaktivitäten auf Basis von HIV-Sequenzen des Subtyps C.

**Tabelle 3: Leserahmen der kodierenden Sequenz von 97cn54**

| Leserahmen | Start | Ende | Start | Ende |
|---|---|---|---|---|
| gag | 167 | 1654 | | |
| pol | 1447 | 4458 | | |
| env | 5589 | 8168 | | |
| vif | 4403 | 4984 | | |
| vpr | 4924 | 5214 | | |
| vpu | 5426 | 5671 | | |
| tat | 5195 | 5409 | 7730 | 7821 |
| rev | 5334 | 5409 | 7730 | 7821 |
| nef | 8170 | 8790 | | |

Die Nummern beziehen sich auf das 5'-Ende der in SEQ ID NO: 1 wiedergegebenen DNA Sequenz.

### Beispiel 15:

### (A) Beschreibung der synthetischen kodierenden Region für C54gp160: C-gp160

Das C-gp120-Gen wurde in die einzigen KpnI/SacI-Restriktionsschnittstellen des pCR-Script amp(+)-Klonierungsvektors (Stratagene, Genbank Accession: U46017) kloniert. Die synthetische, im Kodongebrauch an stark exprimierte Säugergene angepaßte kodierende Region von C54gp160 ist in SEQ ID NO: 3 dargestellt. Die synthetische Signalsequenz kodiert ein Transportsignal für den Import des kodierten Polypeptids in das endiplasmatische Retikulum.

Die Positionen der verschiedenen kodierenden Regionen sind wie folgt:

| CDS | Start | Ende |
|---|---|---|
| synthetische Signalsequenz | 28 | 87 |
| gp160 | 88 | 2580 |

### (B) Beschreibung der synthetischen Sequenz von C54 gagpolnef: C-gpnef

Das Gen C-gpnef wurde in die einzigen KpnI/SacI-Restriktionsschnittstellen des pCR-Script amp(+) Klonierungsvektors (Stratagene) kloniert. Die synthetische, im Kodongebrauch an stark exprimierte Säugergene angepaßte Sequenz von C54gagpolnef ist in SEQ ID NO: 2 dargestellt. In dem vorliegenden Konstrukt wurde das N-terminale Glycin gegen Alanin (Nukleotidsequenz GGC) ausgetauscht, um ein Targeting des Polypeptides an die Zytoplasmamembran und die anschleißende Sekretion von assemblierten Virus-ähnlichen Partikeln via Budding zu vemeiden. Gleichzeitig wurde an der natürlichen Frameshift-Sequenz ein (-1) Leserastersprung eingeführt, der ein obligates Durchlesen der Ribosomen aus dem Gag- in den Pol Leserahmen garantiert und so die Synthese eines GagPolNef Polyproteins sicherstellt.

Die Positionen der verschiedenen kodierenden Regionen sind wie folgt:

| CDS | Start | Ende |
|---|---|---|
| gag | 13 | 1500 |
| 5'pol (ΔRT) | 1501 | 2460 |
| *scrambled* nef | 2461 | 3090 |
| 3'pol (ΔIN) | 3091 | 4155 |
| RT aktives Zentrum | 4156 | 4266 |

### Beispiel 16:

Das durch SEQ ID NO: 1 kodierte GagPolNef Polygen wurde über KpnI/XhoI in den Vektor pcDNA3.1 inseriert und in den E.coli Stamm XL1blue transformiert. Die Fähigkeit des GagPolNef Expressionsvektors eine Gag-spezifische Antikörperantwort zu induzieren wurde in weiblichen BALB/c Mäusen analysiert (Fig. 9). Zwei Gruppen von jeweils 5 Tieren erhielten jeweils eine intramuskuläre (i.m.) Primärimmunisierung von 100 µg DNA pro Immunisierung gefolgt 2 i.m. Folgeimmunisierungen 3 und 6 Wochen später (Gruppe 1: pcDNA-GagPolNef; Gruppe 2: pcDNA). Eine Kontrollgruppe (Gruppe 3) wurde lediglich mit PBS immunisiert. Die Gesamttiter an Gag-spezifischem IgG wurden gegen gereinigtes Gag-Protein im ELISA bestimmt. Die Impfung mit pcDNA-GagPolNef resultierte in einer schnellen Induktion hoher Titer an Gag spezifischen Antikörpern (1:4.000), die gekennzeichnet war durch ein typisches Th1 Profil an Antikörper Isotypen (IgG2a » IgG1). Die beiden Kontrollgruppen 2 und 3 lieferten keine Hinweise auf die Generierung Gag-spezifischer Antikörper. Die Antikörpertiter stiegen beinahe um das hundertfache (1:20.000) 1 Woche nach der ersten Folgeimmunisierung und erreichten Gag-spezifische Endpunkttiter von 1:80,000 eine Woche nach der zweiten Boosterimmunisierung. Zu keinem Zeitpunkt konnte bei den beiden Kontrollgruppen eine signifikante, Gag-spezifische Antikörperantwort nachgewiesen werden.

### Beispiel 17:

Die Antigen-spezifische Zytokinsekretion als Hinweis auf die Induktion einer T-Helfer *Memory-*Antwort wurde aus Milzzellen analysiert, die jeweils 5 Tage nach der zweiten Folgeimmunisierung entnommen wurden. Die Milzzellen der Mäuse, die drei i.m. Immunisierungen mit pcDNA-GagPolNef erhalten hatten, reagierten mit einer deutlichen gIFN Sekretion auf Gag-spezifischen Antigenstimulus (Tabelle 3). Eine vergleichsweise reduzierte gIFN Produktion wurde Milzzellen beobachtet, die aus Mäusen nach dreimaliger subkutaner (s.c.) oder intradermaler (i.d.) Immunisierung mit pcDNA-GagPolNef nach dem selben Schema wie oben gewonnen wurden. In allen Immunisierungsgruppen wurden, unabhängig von der Immunisierungsroute, keine nennenswerte IL4- und IL5 Sekreten aus den spezifisch in vitro restimulierten Milzzellen festgestellt. Eine Zytokinsekretion aus nicht-stimulierten Milzzellen wurde nicht beobachtet.

Die i.m. Immunisierung mit pcDNA-GagPolNef führte demnach zu einem starken Th1 Zytokin-Profil, während die subkutane Verabreichung eher eine schwache Th1 Antwort induzierte.

**Tabelle 4: Zytokin-Profil von in vitro Gag-stimulierten Milzzellen von Mäusen I Immunisierung (Nadelinjektion) oder i.d. bzw. s.c. Immunisierung durch eine Part mit den angegebenen DNA Konstrukten**

| DNA Vakzine | IL-4 (pg/ml) | IL-5 (pg/ml) | IFN-γ (pg/ml) |
|---|---|---|---|
| pcDNA-GagPolNef (i.m.) | <8 | <16 | 3220 ± 840 |
| pcDNA-GagPolNef (i.d.) | <8 | <16 | 80 ± 32 |
| pcDNA-GagPolNef (s.c.) | <8 | <16 | <32 |

| | | | |
|---|---|---|---|
| Mittelwerte ± Standardabweichung von Milzzellen, gewonnen jeweils aus 5 Mäusen pro Experiment | | | |

### Beispiel 18:

Um die Fähigkeit von pcDNA-GagPolNef zur Induktion Gag-spezifischer CTLs zu überprüfen wurden Milzzellen 3 Wochen nach einer primären Immunisierung mit pcDNA-GagPolNef (Gruppe 1), pcDNA (Gruppe 2) und PBS (Gruppe 3) *in vitro* in einer gemischen-Lymphozyten-Tumor-Zellkultur für 6 Tage spezifisch restimuliert und anschließend hinsichtlich ihrer zytotoxischen Aktivität untersucht. Bei dem nonameren, vom Gag Protein der Subtyp B-Viren (IIIB-Isolat) abgeleiteten AMQMLKETI Peptid (Einbuchstabencode), das in diesem Versuch zur *in vitro* Restimulation gleichwie zur Bestimmung der spezifischen zytotoxischen Aktivität eingestzt wurde, stellt bekannterweise ein D^{d}-restringiertes CTL Epitope in der BALB/c Maus dar. *Gag*-spezifische zytotoxische T-Zellen konnten nach einer einmaligen i.m. Injektion mit dem pcDNA-GagPolNef Plasmid, nicht jedoch in einer der beiden Kontrollgruppen 2 und 3 festgestellt werden. Die Behandlung von Milzzellen mit dem oben genannten Peptid resultierte nicht in einem *in vitro Priming* Gag-spezifischer zytotoxischer T-Zellen. Diese Ergebnisse bestätigen (i) die Fähigkeit von pcDNA-GagPolNef zur Induktion Spezifischer zytotoxischer T-Zellen, die (ii) Subtyp-übergreifend aktiv sind (Figur 9).

### Literatur

Bai, X., Su, L., Zhang, Y., and et al(1997). Subtype and sequence analysis of the C2V3 region of gp120 gene among HIV-1 strains in Xinjiang. Chin. J. Virology 13.
Carr, J. K., Salminen, M. O., Koch, C., Gotte, D., Artenstein, A. W., Hegerich, P. A., St Louis, D., Burke, D. S., and McCutchan, F. E.(1996). Full-length sequence and mosaic structure of a human immunodeficiency virus type 1 isolate from Thailand. J. Virol. 70, 5935-5943.
Carr, J. K., Salminen, M. O., Albert, J., Sanders Buell, E., Gotte, D., Birx, D. L., and McCutchan, F. E.(1998). Full genome sequences of human immunodeficiency virus type 1 subtypes G and A/G intersubtype recombinants. Virology 247, 22-31.
Esparza, J., Osmanov, S., and Heyward, W. L.(1995). HIV preventive vaccines. Progress to date. Drugs 50, 792-804.
Expert group of joint United Nations programme on HIV/AIDS(1999). Implications of HIV variability for transmission: scientific and policy issues. AIDS 11, UNAIDS 1-UNAIDS 15.
Gao, F., Robertson, D. L., Morrison, S. G., Hui, H., Craig, S., Decker, J., Fultz, P. N., Girard, M., Shaw, G. M., Hahn, B. H., and Sharp, P. M.(1996). The heterosexual human immunodeficiency virus type 1 epidemic in Thailand is caused by an intersubtype (A/E) recombinant of African origin. J. Virol. 70, 7013-7029.
Gao, F., Robertson, D. L., Carruthers, C. D., Morrison, S. G., Jian, B., Chen, Y., Barre Sinoussi, F., Girard, M., Srinivasan, A., Abimiku, A. G., Shaw, G. M., Sharp, P. M., and Hahn, B. H.(1998). A comprehensive panel of near-full-length clones and reference sequences for nonsubtype B isolates of human immunodeficiency virus type 1. J. Virol. 72, 5680-5698.
Gaywee, J., Artenstein, A. W., VanCott, T. C., Trichavaroj, R., Sukchamnong, A., Amlee, P., de Souza, M., McCutchan, F. E., Carr, J. K., Markowitz, L. E., Michael, R., and Nittayaphan, S.(1996). Correlation of genetic and serologic approaches to HIV-1 subtyping in Thailand. J. Acquir. Immune. Defic. Syndr. Hum. Retrovirol. 13, 392-396.
Graf, M., Shao, Y., Zhao, Q., Seidl, T., Kostler, J., Wolf, H., and Wagner, R.(1998). Cloning and characterization of a virtually full-length HIV type 1 genome from a subtype B'-Thai strain representing the most prevalent B-clade isolate in China. AIDS Res. Hum. Retroviruses 14, 285-288.
Graham, B. S. and Wright, P. F.(1995). Candidate AIDS vaccines. N. Engl. J. Med. 333, 1331-1339.
Kostrikis, L. G., Bagdades, E., Cao, Y., Zhang, L., Dimitriou, D., and Ho, D. D.(1995). Genetic analysis of human immunodeficiency virus type 1 strains from patients in Cyprus: identification of a new subtype designated subtype I. J. Virol. 69, 6122-6130.
Leitner, T. and Albert, J.(1995). Human Retroviruses and AIDS 1995: a compilation and analysis of nucleic acid and amino acid sequences. (Myers, G., Korber, B., Wain-Hobson, S., Jeang, K., Mellors, J., McCutchan, F., Henderson, L., and Pavlakis, G. Eds.) Los Alamos National Laboratory, Los Alamos, N. Mex. III147-III150.
Lole, K. S., Bollinger, R. C., Paranjape, R. S., Gadkari, D., Kulkarni, S. S., Novak, N. G., Ingersoll, R., Sheppard, H. W., and Ray, S. C.(1999). Full-length human immunodeficiency virus type 1 genomes from subtype C-infected seroconverters in India, with evidence of intersubtype recombination. J. Virol. 73, 152-160.
Loussert Ajaka, I., Chaix, M. L., Korber, B., Letourneur, F., Gomas, E., Allen, E., Ly, T. D., Brun Vezinet, F., Simon, F., and Saragosti, S.(1995). Variability of human immunodeficiency virus type 1 group O strains isolated from Cameroonian patients living in France. . J. Virol. 69, 5640-5649.
Luo, C. C., Tian, C., Hu, D. J., Kai, M., Dondero, T., and Zheng, X.(1995). HIV-1 subtype C in China [letter]. Lancet 345, 1051-1052.
Myers, G., Korber, B., Foley, B., Jeang, K. T., Mellors, J. W., and Wain Hobson, S.(1996). Human retroviruses and AIDS: a compilation and analysis of nucleic acid and amino acid sequences*.* (Anonymous Theoretical Biology and Biophysics Group, Los Alamos, N. Mex. Salminen, M. O., Koch, C., Sanders Buell, E., Ehrenberg, P. K., Michael, N. L., Carr, J. K., Burke, D. S., and McCutchan, F. E.(1995). Recovery of virtually full-length HIV-1 provirus of diverse subtypes from primary virus cultures using the polymerase chain reaction. Virology 213, 80-86.
Shao, Y., Zhao, Q., Wang B., and et al(1994). Sequence analysis of HIV env gene among HIV infected IDUs in Yunnan epidemic area of China. Chin. J. Virology 10, 291-299.
Shao, Y., Su, L., Sun, X., and et al(1998). Molecular Epidemiology of HIV infection in China. 12th world AIDS conference, Geneva 13132**,** (Abstract).
Shao, Y., Guan, Y., Zhao, Q., and et al(1999). Genetic variation and molecular epidemiology of the Ruily HIV-1 strains of Yunnan in 1995. Chin. J. Virol. 12, 9.
Sharp, P. M., Robertson, D. L., and Hahn, B. H.(1995). Cross-species transmission and recombination of 'AIDS' viruses. Philos. Trans. R. Soc. Lond. B. Biol. Sci. 349, 41-47.
Sharp, P. M., Bailes, E., Robertson, D. L., Gao, F., and Hahn, B. H.(1999). Origins and evolution of AIDS viruses. Biol. Bull. 196, 338-342.
World Health Organisation Network for HIV Isolation and Characterization(1994). HIV-1 variation in WHO-sponsored vaccine-evaluation sites:genetic screening, sequence analysis and preliminary biological characterization of selected viral strains. AIDS Res. Hum. Retroviruses 10, 1327-1344.
Yu, H., Su, L., and Shao, Y.(1997). Identification of the HIV-1 subtypes by HMA and sequencing. Chin. J. Epidemiol. 18, 201-204.

## Patentansprüche

1. Ein Polynukleotid mit der Nukleinsäuresequenz gemäß SEQ ID NO:1, 2 oder 3 oder einer Sequenz von den Nukleotidnummern 167 bis 1654, 1447 bis 4458, 5589 bis 8168, 4403 bis 4984, 4924 bis 5214, 5426 bis 5671, 8170 bis 8790, 5195 bis 5409, 7730 bis 7821, 5334 bis 5409 oder 7730 bis 7821, oder eine auf einem Polynukleotid zusammenhängende Sequenz von 5195 bis 5409 und 7730 bis 7821, oder 5334 bis 5409 und 7730 bis 7821, jeweils auf die SEQ ID NO:1 bezogen.

2. Polynukleotid mit mehr als einer kontinuierlichen Nukleinsäuresequenz gemäß Anspruch 1.

3. Polynukleotid nach Anspruch 2, wobei mindestens zwei der kontinuierlichen Nukleinsäuresequenzen durch einen Nukleotid-Platzhalter ("spacer") getrennt sind.

4. Codon-optimiertes Polynukleotid kodierend ein Gag-Polypeptid, wie es durch die Nukleinsäuresequenz von Nukleotidnummer 167 bis 1654 kodiert wird, ein Pol-Polypeptid, wie es durch die Nukleinsäuresequenz von Nukleotidnummer 1447 bis 4458 kodiert wird, ein env-Polypeptid, wie es durch die Nukleinsäuresequenz von Nukleotidnummer 5589 bis 8168 kodiert wird, ein vif-Polypeptid, wie es durch die Nukleinsäuresequenz von Nukleotidnummer 4403 bis 4984 kodiert wird, ein vpr-Polypeptid, wie es durch die Nukleinsäuresequenz von Nukleotidnummer 4924 bis 5214 kodiert wird, ein vpu-Polypeptid, wie es durch die Nukleinsäuresequenz von Nukleotidnummer 5426 bis 5671 kodiert wird, ein nef-Polypeptid, wie es durch die Nukleinsäuresequenz von Nukleotidnummer 8170 bis 8790 kodiert wird, ein rev-Polypeptid, wie es durch die Nukleinsäuresequenz von Nukleotidnummer 5334 bis 5409 und Nukleotidnummer 7730 bis 7821 kodiert wird, wobei die Nukleotidnummern jeweils auf die SEQ ID NO: 1 bezogen sind,
ein gag-Polypeptid, wie es durch die Nukleinsäuresequenz von Nukleotidnummer 13 bis 1500 kodiert wird, ein 5'pol-Polypeptid, wie es durch die Nukleinsäuresequenz von Nukleotidnummer 1501 bis 2460 kodiert wird, ein *scrambeled* nef-Polypeptid, wie es durch die Nukleinsäuresequenz von Nukleotidnummer 2461 bis 3090 kodiert wird, ein 3'pol-Polypeptid, wie es durch die Nukleinsäuresequenz von Nukleotidnummer 3091 bis 4155 kodiert wird und/oder ein RT-aktives Zentrum-Polypeptid, wie es durch die Nukleinsäuresequenz von Nukleotidnummer 4156 bis 4266 kodiert wird, wobei die Nukleotidnummern jeweils auf die SEQ ID NO:2 bezogen sind.

5. DNA-, bakterielle oder virale Vektoren, umfassend das Polynukleotid nach einem der Ansprüche 1 bis 4.

6. Polynukleotid nach einem der Ansprüche 1 bis 4 als Arzneimittel, Impfstoff oder Diagnostikum, oder ein Polynukleotid mit einer oder mehreren mindestens 27 Nukleotide langen Fragmenten eines oder mehrerer Polynukleotide nach einem der Ansprüche 1 bis 4 als Arzneimittel, Impfstoff oder Diagnostikum, oder ein Polynukleotid mit zwei oder mehreren mindestens 27 Nukleotide langen Fragmenten getrennt durch Nukleotid-Platzhalter ("spacer") eines oder mehrerer Polynukleotide nach einem der Ansprüche 1 bis 4 als Arzneimittel, Impfstoff oder Diagnostikum.

7. Verwendung des Polynukleotids nach einem der Ansprüche 1 bis 4, oder eines Polynukleotids mit einer oder mehreren mindestens 27 Nukleotide langen Fragmenten eines oder mehrerer Polynukleotide nach einem der Ansprüche 1 bis 4 oder eines Polynukleotids mit zwei oder mehreren mindestens 27 Nukleotide langen Fragmenten getrennt durch Nukleotid-Platzhalter ("spacer") eines oder mehrerer Polynukleotide nach einem der Ansprüche 1 bis 4 zur Herstellung eines Arzneimittels oder Impfstoffs für die Behandlung oder Prävention oder eines Diagnostikums zur Diagnose von HIV-Infektionen.

8. Gag-Polypeptid, kodiert durch die Nukleinsäuresequenz von Nukleotidnummer 167 bis 1654, Pol-Polypeptid, kodiert durch die Nukleinsäuresequenz von Nukleotidnummer 1447 bis 4458, env-Polypeptid, kodiert durch die Nukleinsäuresequenz von Nukleotidnummer 5589 bis 8168, vif-Polypeptid, kodiert durch die Nukleinsäuresequenz von Nukleotidnummer 4403 bis 4984, vpr-Polypeptid, kodiert durch die Nukleinsäuresequenz von Nukleotidnummern 4924 bis 5214, vpu-Polypeptid, kodiert durch die Nukleinsäuresequenz von Nukleotidnummer 5426 bis 5671, nef-Polypeptid, kodiert durch die Nukleinsäuresequenz von Nukleotidnummer 8170 bis 8790, rev-Polypeptid, kodiert durch die Nukleinsäuresequenz von Nukleotidnummer 5334 bis 5409 und Nukleotidnummer 7730 bis 7821, jeweils auf die SEQ ID NO:1 bezogen,
gag-Polypeptid, kodiert durch die Nukleinsäuresequenz von Nukleotidnummer 13 bis 1500, 5'pol-Polypeptid, kodiert durch die Nukleinsäuresequenz von Nukleotidnummer 1501 bis 2460, *scrambled* nef-Polypeptid, kodiert durch die Nukleinsäuresequenz von Nukleotidnummer 2461 bis 3090, 3'pol-Polypeptid, kodiert durch die Nukleinsäuresequenz von Nukleotidnummer 3091 bis 4155 oder RT-aktives Zentrum-Polypeptid, kodiert durch die Nukleinsäuresequenz von Nukleotidnummer 4156 bis 4266, jeweils auf die SEQ ID NO:2 bezogen, oder ein Fragment der Polypeptide, das mindestens 9 Aminosäuren lang ist.

9. Polypeptid nach Anspruch 8, mit mehr als einem kontinuierlichen mindestens 9 Aminosäuren langen Fragment.

10. Polypeptid nach Anspruch 9, wobei mindestens zwei der Fragmente durch einen Aminosäure-Platzhalter getrennt sind.

11. Polypeptid nach einem der Ansprüche 8 bis 10, wobei die Aminosäuresequenz dem HIV-Hüllprotein oder einem Fragment des HIV-Hüllproteins entspricht.

12. Polypeptid nach einem der Ansprüche 8 bis 11, ferner umfassend ein B-Zellepitop, ein MHC Klasse II-restringiertes T-Helferepitop, ein MHC Klasse I-restringiertes zytotoxisches T-Zellepitop oder eine Kombinationen davon.

13. Polypeptid nach Anspruch 12, wobei das B-Zellepitop ein Konformations-Epitop oder ein lineares Epitop und das MHC Klasse II-restringierte T-Helferepitop oder das MHC Klasse I-restringierte zytotoxische T-Zellepitop ein lineares Epitop ist.

14. Das Polypeptid nach einem der Ansprüche 8 bis 13 als Arzneimittel, Impfstoff oder Diagnostikum.

15. Verwendung des Polypeptids nach einem der Ansprüche 8 bis 13 zur Herstellung eines Arzneimittels oder Impfstoffs für die Behandlung oder Prävention oder eines Diagnostikums zur Diagnose von HIV-Infektionen.

16. Isoliertes Polypeptid, das spezifisch ein Polypeptid nach einem der Ansprüche 8 bis 13 bindet, wobei das isolierte Polypeptid ein Antikörper, Antikörperderivat oder ein Derivat des menschlichen pankreatischen sekretorischen Trypsin-Inhibitors (hPSTI) ist.

17. Isoliertes Polypeptid nach Anspruch 16 als Arzneimittel oder Diagnostikum.

18. Verwendung des isolierten Polypeptids nach Anspruch 16 oder 17 zur Herstellung eines Arzneimittels für die Behandlung oder Prävention oder eines Diagnostikums zur Diagnose von HIV-Infektionen.

19. Säugerzelle, transformiert mit einem Polynukleotid mit einer Nukleinsäuresequenz gemäß SEQ ID NO:1 und/oder 3 oder einem codon-optimierten Polynukleotid, wie es in Anspruch 4 definiert ist.

## Claims

1. A polynucleotide having the nucleic acid sequence according to SEQ ID NO:1, 2 or 3 or a sequence from the nucleotide numbers 167 to 1654, 1447 to 4458, 5589 to 8168, 4403 to 4984, 4924 to 5214, 5426 to 5671, 8170 to 8790, 5195 to 5409, 7730 to 7821, 5334 to 5409 or 7730 to 7821, or a continuous sequence on a polynucleotide from the nucleotide numbers 5195 to 5409 and 7730 to 7821, or 5334 to 5409 and 7730 to 7821, each referring to SEQ ID NO:1.

2. The polynucleotide having more than one continuous nucleic acid sequence according to claim 1.

3. The polynucleotide according to claim 2, wherein at least two of the continuous nucleic acid sequences are separated by a nucleotide sequence spacer.

4. Codon-optimized polynucleotide encoding a gag-polypeptide as encoded by the nucleic acid sequence from the nucleotide number 167 to 1654, a pol-polypeptide as encoded by the nucleic acid sequence from the nucleotide number 1447 to 4458, a env-polypeptide as encoded by the nucleic acid sequence from the nucleotide number 5589 to 8168, a vif-polypeptide as encoded by the nucleic acid sequence from the nucleotide number 4403 to 4984, a vpr-polypeptide as encoded by the nucleic acid sequence from the nucleotide number 4924 to 5214, a vpu-polypeptide as encoded by the nucleic acid sequence from the nucleotide number 5426 to 5671, a nef-polypeptide as encoded by the nucleic acid sequence from the nucleotide number 8170 to 8790, a rev-polypeptide as encoded by the nucleic acid sequence from the nucleotide number 5334 to 5409 and the nucleotide number 7730 to 7821, wherein the nucleotide numbers each refer to SEQ ID NO:1,
a gag-polypeptide as encoded by the nucleic acid sequence from the nucleotide number 13 to 1500, a 5'pol-polypeptide as encoded by the nucleic acid sequence from the nucleotide number 1501 to 2460, a scrambled nef-polypeptide as encoded by the nucleic acid sequence from the nucleotide number 2461 to 3090, a 3'pol-polypeptide as encoded by the nucleic acid sequence from the nucleotide number 3091 to 4155 and/or a RT-active site polypeptide as encoded by the nucleic acid sequence from the nucleotide number 4156 to 4266, wherein the nucleotide numbers each refer to SEQ ID NO:2.

5. DNA, bacterial or viral vectors, comprising the polynucleotide according to any one of claims 1 to 4.

6. The polynucleotide according to any one of claims 1 to 4 as a medicament, vaccine or diagnostic substance, or a polynucleotide having one or more at least 27 nucleotide long fragment(s) of one or more polynucleotide(s) according to any one of claims 1 to 4 as a medicament, vaccine or diagnostic substance, or a polynucleotide having two or more at least 27 nucleotide long fragments separated by nucleotide sequence spacer of one or more polynucleotide(s) according to any one of claims 1 to 4 as a medicament, vaccine or diagnostic substance.

7. Use of the polynucleotide according to any one of claims 1 to 4, or of a polynucleotide having one or more at least 27 nucleotide long fragment(s) of one or more polynucleotide(s) according to any one of claims 1 to 4, or of a polynucleotide having two or more at least 27 nucleotide long fragments separated by nucleotide sequence spacer of one or more polynucleotide(s) according to any one of claims 1 to 4, for the manufacture of a medicament or vaccine for the treatment or prevention of HIV infections, or of a diagnostic substance for the diagnosis of HIV infections.

8. Gag-polypeptide, encoded by the nucleic acid sequence of nucleotide number 167 to 1654, pol-polypeptide encoded by the nucleic acid sequence from the nucleotide number 1447 to 4458, env-polypeptide encoded by the nucleic acid sequence from the nucleotide number 5589 to 8168, vif-polypeptide encoded by the nucleic acid sequence from the nucleotide number 4403 to 4984, vpr-polypeptide encoded by the nucleic acid sequence from the nucleotide number 4924 to 5214, vpu-polypeptide encoded by the nucleic acid sequence from the nucleotide number 5426 to 5671, nef-polypeptide encoded by the nucleic acid sequence from the nucleotide number 8170 to 8790, rev-polypeptide encoded by the nucleic acid sequence from the nucleotide number 5334 to 5409 and nucleotide number 7730 to 7821, each refer to the SEQ ID NO:1,
gag-polypeptide encoded by the nucleic acid sequence from the nucleotide number 13 to 1500, 5'pol-polypeptide encoded by the nucleic acid sequence from the nucleotide number 1501 to 2460, scrambled nef-polypeptide encoded by the nucleic acid sequence from the nucleotide number 2461 to 3090, 3'pol-polypeptide encoded by the nucleic acid sequence from the nucleotide number 3091 to 4155 or RT-active site polypeptide encoded by the nucleic acid sequence of nucleotide number 4156 to 4266, each refer to SEQ ID NO:2, or a fragment of the polypeptides, which is at least 9 amino acids long.

9. The polypeptide according to claim 8, having more than one continuous fragment of at least 9 amino acids.

10. The polypeptide according to claim 9, wherein at least two fragments are separated by an amino acid spacer.

11. The polypeptide according to any one of claims 8 to 10, said amino acid sequence corresponds to the HIV envelope protein or a fragment thereof..

12. The polypeptide according to any one of claims 8 to 11, further comprising a B cell epitope, an MHC class II restricted T helper epitope, an MHC class I restricted cytotoxic T cell epitope or a combination thereof.

13. The polypeptide according to claim 12, wherein said B cell epitope is a conformational epitope or a linear epitope, and said MHC class II restricted T helper epitope or said MHC class I restricted cytotoxic T cell epitope is a linear epitope.

14. The polypeptide according to any one of claims 8 to 13 as a medicament, vaccine or diagnostic substance.

15. Use of the polypeptide according to any one of claims 8 to 13 for the manufacture of a medicament or vaccine for the treatment or prevention of HIV infections, or of a diagnostic substance for the diagnosis of HIV infections.

16. Isolated polypeptide specifically binding a polypeptide according to any one of claims 8 to 13, wherein isolated polypeptide is an antibody, antibody derivative or a derivative of the human pancreatic secretory trypsine inhibitor (hPSTI).

17. The isolated polypeptide according to claim 16 as a medicament or diagnostic substance.

18. Use of the isolated polypeptide according to any one of claims 16 to 17 for the manufacture of a medicament for the treatment or prevention of HIV infections, or of a diagnostic substance for the diagnosis of HIV infections.

19. Mammalian cell transformed with a polynucleotide having a nucleic acid sequence according to SEQ ID NO:1 and/or 3, or a codon-optimized polynucleotide as defined in claim 4.

## Revendications

1. Polynucléotide ayant la séquence d'acide nucléique selon la SEQ ID N°1, 2 ou 3 ou une séquence des nucléotides numéros 167 à 1654, 1447 à 4458, 5589 à 8168, 4403 à 4984, 4924 à 5214, 5426 à 5671, 8170 à 8790, 5195 à 5409, 7730 à 7821, 5334 à 5409 ou 7730 à 7821, ou une séquence relative à un polynucléotide de 5195 à 5409 et 7730 à 7821, ou 5334 à 5409 et 7730 à 7821, respectivement par rapport à la SEQ ID N°1.

2. Polynucléotide ayant plus d'une séquence d'acide nucléique continue selon la revendication 1.

3. Polynucléotide selon la revendication 2, dans lequel au moins deux des séquences d'acide nucléique continues sont séparées par un espaceur nucléotidique (« spacer »).

4. Polynucléotide à codon(s) optimisé(s) codant un polypeptide gag, tel qu'il est codé par la séquence d'acide nucléique des nucléotides numéros 167 à 1654, un polypeptide pol, tel qu'il est codé par la séquence d'acide nucléique des nucléotides numéros 1447 à 4458, un polypeptide env, tel qu'il est codé par la séquence d'acide nucléique des nucléotides numéros 5589 à 8168, un polypeptide vif, tel qu'il est codé par la séquence d'acide nucléique des nucléotides numéros 4403 à 4984, un polypeptide vpr, tel qu'il est codé par la séquence d'acide nucléique des nucléotides numéros 4924 à 5214, un polypeptide vpu, tel qu'il est codé par la séquence d'acide nucléique des nucléotides numéros 5426 à 5671, un polypeptide nef, tel qu'il est codé par la séquence d'acide nucléique des nucléotides numéros 8170 à 8790, un polypeptide rev, tel qu'il est codé par la séquence d'acide nucléique des nucléotides numéros 5334 à 5409 et nucléotides numéros 7730 à 7821, les numéros des nucléotides se rapportant respectivement à la SEQ ID N°1,
un polypeptide gag, tel qu'il est codé par la séquence d'acide nucléique des nucléotides numéros 13 à 1500, un polypeptide 5'pol, tel qu'il est codé par la séquence d'acide nucléique des nucléotides numéros 1501 à 2460, un polypeptide nef *scrambeled,* tel qu'il est codé par la séquence d'acide nucléique des nucléotides numéros 2461 à 3090, un polypeptide 3'pol, tel qu'il est codé par la séquence d'acide nucléique des nucléotides numéros 3091 à 4155, et/ou un polypeptide central RT-actif, tel qu'il est codé par la séquence d'acide nucléique des nucléotides numéros 4156 à 4266, les numéros des nucléotides se rapportant respectivement à la SEQ ID N°2.

5. Vecteurs ADN, bactériens ou viraux, comprenant le polynucléotide selon l'une des revendications 1 à 4.

6. Polynucléotide selon l'une des revendications 1 à 4 à titre de médicament, vaccin ou élément diagnostique, ou polynucléotide ayant un ou plusieurs fragments, longs d'au moins 27 nucléotides, d'un ou de plusieurs polynucléotides selon l'une des revendications 1 à 4 à titre de médicament, vaccin ou élément diagnostique, ou polynucléotide ayant deux ou plusieurs fragments, longs d'au moins 27 nucléotides, séparés par des espaceurs nucléotidiques (« spacer ») d'un ou de plusieurs polynucléotides selon l'une des revendications 1 à 4 à titre de médicament, vaccin ou élément diagnostique.

7. Utilisation du polynucléotide selon l'une des revendications 1 à 4, ou d'un polynucléotide ayant un ou plusieurs fragments, longs d'au moins 27 nucléotides, d'un ou de plusieurs polynucléotides selon l'une des revendications 1 à 4 ou d'un polynucléotide ayant deux ou plusieurs fragments, longs d'au moins 27 nucléotides, séparés par des espaceurs nucléotidiques (« spacer ») d'un ou de plusieurs polynucléotides selon l'une des revendications 1 à 4 pour la fabrication d'un médicament ou d'un vaccin pour le traitement ou la prévention ou d'un élément diagnostique pour le diagnostic des infections au VIH.

8. Polypeptide gag, codé par la séquence d'acide nucléique des nucléotides numéros 167 à 1654, un polypeptide pol, codé par la séquence d'acide nucléique des nucléotides numéros 1447 à 4458, un polypeptide env, codé par la séquence d'acide nucléique des nucléotides numéros 5589 à 8168, un polypeptide vif, codé par la séquence d'acide nucléique des nucléotides numéros 4403 à 4984, un polypeptide vpr, codé par la séquence d'acide nucléique des nucléotides numéros 4924 à 5214, un polypeptide vpu, codé par la séquence d'acide nucléique des nucléotides numéros 5426 à 5671, un polypeptide nef, codé par la séquence d'acide nucléique des nucléotides numéros 8170 à 8790, un polypeptide rev, codé par la séquence d'acide nucléique des nucléotides numéros 5334 à 5409 et des nucléotides numéros 7730 à 7821, se rapportant respectivement à la SEQ ID N°1,
un polypeptide gag, codé par la séquence d'acide nucléique des nucléotides numéros 13 à 1500, un polypeptide 5'pol, codé par la séquence d'acide nucléique des nucléotides numéros 1501 à 2460, un polypeptide nef *scrambeled,* codé par la séquence d'acide nucléique des nucléotides numéros 2461 à 3090, un polypeptide 3'pol, codé par la séquence d'acide nucléique des nucléotides numéros 3091 à 4155, ou un polypeptide central RT-actif, codé par la séquence d'acide nucléique des nucléotides numéros 4156 à 4266, se rapportant respectivement à la SEQ ID N°2, ou un fragment des polypeptides, qui a une longueur d'au moins 9 acides aminés.

9. Polypeptide selon la revendication 8, ayant plus d'un fragment continu long d'au moins 9 acides aminés.

10. Polypeptide selon la revendication 9, dans lequel au moins deux des fragments sont séparés par un espaceur acide aminé.

11. Polypeptide selon l'une des revendications 8 à 10, dans lequel la séquence d'acide aminé correspond à la protéine d'enveloppe du VIH ou un fragment de la protéine d'enveloppe du VIH.

12. Polypeptide selon l'une des revendications 8 à 11, comprenant en outre un épitope de cellules B, un épitope de cellules T-helper restreint par le CMH de classe II, un épitope de cellules T cytotoxiques restreint par le CMH de classe I ou une combinaison de ceux-ci.

13. Polypeptide selon la revendication 12, où l'épitope de cellules B est un épitope conformationnel ou un épitope linéaire et l'épitope de cellules T-helper restreint par le CMH de classe II ou l'épitope de cellules T cytotoxiques restreint par le CMH de classe I est un épitope linéaire.

14. Polypeptide selon l'une des revendications 8 à 13 à titre de médicaments, vaccin ou élément diagnostique.

15. Utilisation du polypeptide selon l'une des revendications 8 à 13 pour la fabrication d'un médicament ou d'un vaccin pour le traitement ou la prévention ou d'un élément diagnostique pour le diagnostic des infections au VIH.

16. Polypeptide isolé qui lie spécifiquement un polypeptide selon l'une des revendications 8 à 13, où le polypeptide isolé est un anticorps, un dérivé d'anticorps ou un dérivé de l'inhibiteur de trypsine sécrétoire pancréatique humaine (hPSTI).

17. Polypeptide isolé selon la revendication 16 à titre de médicament ou élément diagnostic.

18. Utilisation du polypeptide isolé selon la revendication 16 ou 17 pour la fabrication d'un médicament pour le traitement ou la prévention ou d'un élément diagnostique pour le diagnostic des infections au VIH.

19. Cellules de mammifères, transformées avec un polynucléotide ayant une séquence d'acide nucléique selon la SEQ ID N°1 et/ou 3 ou un polynucléotide à codon(s) optimisé(s), tel qu'il est défini dans la revendication 4.
